# EUROPEAN PATENT APPLICATION

(11) **EP 3 650 036 A1**
(43) Date of publication of application: **13.05.2020**
(21) Application number: 18306454.2
(22) Date of filing: 06.11.2018
(51) Int. Cl.: A61K 38/08, A61P 35/00, A61P 35/02

(54) **SYNTHETIC PEPTIDES INDUCING IMMUNOGENIC CELL DEATH**

(71) Applicant: SORBONNE UNIVERSITE, 75006 Paris (FR); CENTRE NATIONAL DE LA RECHERCHE SCIENTIFIQUE, 75016 Paris (FR)
(72) Inventor: Karoyan, Philippe, 91680 Courson-Monteloup (FR); Martinez Torres, Ana Carolina, 66455 San Nicolas de los Garza (MX); Rodriguez Padilla, Maria Cristina, 66455 San Nicolas de los Garza (MX)
(74) Representative: Gevers & Orès

(57) **Abstract**

The invention relates to TSP-1-derived peptides capable of inducing immunogenic cell death, in particular immunogenic cancer cell death. It further relates to uses of such peptides, in particular to prepare a pharmaceutical composition to allow or improve the efficiency of a therapy of cancer in a subject in need thereof.

## Description

The invention relates to TSP-1-derived peptides capable of inducing immunogenic cell death, in particular immunogenic cancer cell death. It further relates to uses of such peptides, in particular to prepare a pharmaceutical composition to allow or improve the efficiency of a therapy of cancer in a subject in need thereof.

Immunogenic cell death (ICD) is a type of regulated cell death that activates an adaptive immune response against dead-cell-associated antigens, inducing tumor cell immunogenicity (1,2). ICD is characterized by the exposure or release of endogenous immunogenic biomolecules, namely damage-associated molecular patterns (DAMPs) (3). In physiological conditions, DAMPs are inside the cells, but when exposed or released, in case of stress, injury, or cell death, they bind receptors on immune cells (4,5). The main DAMPs related to ICD exposed at the cell surface and/or released to the extracellular media are calreticulin (CRT) (6-8) and other endoplasmic reticulum (ER) proteins like heatshock protein 70 and 90 (HSP70 and HSP90) (9,10), and secretion of ATP (11-13) and the non-histone chromatin protein high-mobility group box 1 (HMGB1) (14,15). Collectively, these DAMPs recruit antigen-presenting cells (APCs) to ICD sites and stimulate the uptake, processing, and presentation of dead-cell-associated antigens, resulting in an adaptive immune response (1,16-18).

A subset of chemotherapeutic agents including doxorubicin, mitoxantrone, oxaliplatin, bortezomib, cyclophosphamide, and anthracycline has the ability to trigger ICD (18,21), hence activating anticancer immune responses (1). These drugs are used to treat different types of cancer including hematological malignancies like acute lymphoblastic leukemia (ALL).

Previous reports using other ICD inducers also prevented tumor growth: such is the case of melphalan, alkylating agent, used in melanoma treatment, where C57BL6 mice were injected with melphalan killed murine B78 melanoma cells and re-challenged 10 days later with B78 viable cells obtaining 40% of mice without tumor (51). Similar results were obtained using doxorubicin in mouse colon carcinoma (CT26) cell line (42). The use of this vaccine helps to stimulate anti-cancer immunity through the maturation of DCs and cytotoxic T cell activation (52) as well as enhancing NK cytotoxic activity (53).

Immunotherapy is a promising treatment option against cancer (54), using host immune defenses against cancer and seeking to endow cancer cells with immunogenicity (55). The increased immunogenicity of tumor cells triggers the antitumor immune responses which could offer long-term therapeutic effects (1). The finding that certain drugs are able to induce the awakening of the immune response by releasing DAMPs and generating ICD, triggered the investigations that look for these type of agents (1,42,53,56). Anthracyclines, platinum derivatives, alkylating agents, and proteasome inhibitors are some chemotherapeutic drugs with vast evidence on triggering ICD (57). Other therapeutic modalities that display ICD induction are photodynamic therapy (58), radiotherapy (59), oncolytic viruses (60,61), high hydrostatic pressure (62) and other phytochemical agents such as shikonin (63,64) and capsaicin (65,66).

The development of new treatments able to stimulate the immune system, such as ICD-inducers, remains important to fight chemoresistant malignancies.

Inventors have now evidenced that a known family of synthetic peptides is able to induce a selective immunogenic cell death (ICD) in cancer cell lines.

Said synthetic peptides have been previously described as CD-47 agonists able to trigger Programmed Cell Death (PCD) and thus treat diseases associated with defects in PCD (WO2017194634, WO2017194627).

Inventors have indeed obtained data that indicate that PKHB1 induces caspase-independent and calcium-dependent cell death in leukemic cells while sparing non-tumor murine and human cells. Moreover, these results show that PKHB1 can induce ICD in leukemic cells as it induces CRT exposure and DAMPs release, *in vitro,* and prophylactic vaccinations inhibit tumor establishment *in vivo.*

The present invention thus relates to synthetic TSP-1-derived peptide for its use to induce immunogenic cell death in the treatment of cancer.

The present invention also relates to the use of synthetic TSP-1-derived peptide for the preparation of a pharmaceutical composition for inducing immunogenic cell death in the treatment of cancer.

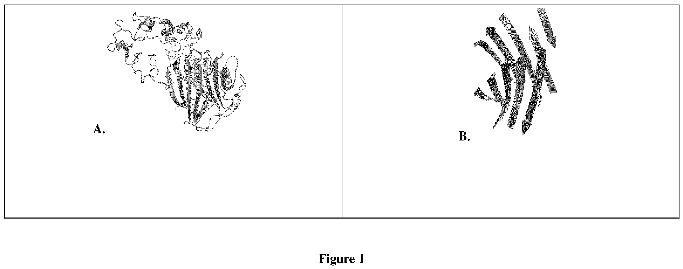

According to the present invention, synthetic TSP-1-derived peptides are selected amongst those mimicking the beta strand number 7 of TSP-1 or the beta-sheet formed by the association of beta strands number 7 and number 8 of TSP-1, as depicted on **Figure 1****.**

According to one embodiment, synthetic TSP-1-derived peptide is a compound or a pharmaceutical acceptable salt thereof comprising a hexapeptide sequence of formula (I):

-X₁-X₂-X₃-X₄-X₅-X₆- (I)

wherein:
- X₁, X₂, X₃, X₄, X₅, X₆ are independently linked to each other according to formula (I) via peptide bonds or at least one pseudopeptide bond;
- X₁ is a residue chosen in the list consisting of substituted or unsubstituted phenylalanine, substituted or unsubstituted para-tyrosine, substituted or unsubstituted ortho-tyrosine, substituted or unsubstituted meta-tyrosine, or substituted or unsubstituted homo-phenylalanine;
- X₂ is a residue chosen in the list consisting of substituted or unsubstituted para- tyrosine, substituted or unsubstituted ortho-tyrosine, substituted or unsubstituted meta- tyrosine, substituted or unsubstituted phenylalanine, homo-phenylalanine, homo-meta- tyrosine, homo-para-tyrosine or homo-ortho-tyrosine;
- X₃ is a residue chosen in the list consisting of substituted or unsubstituted valine, substituted or unsubstituted alanine, substituted or unsubstituted leucine, substituted or unsubstituted isoleucine, preferably valine;
- X₄ is a residue chosen in the list consisting of substituted or unsubstituted valine, substituted or unsubstituted alanine, substituted or unsubstituted leucine, substituted or unsubstituted isoleucine, preferably valine;
- X₅ is a residue chosen in the list consisting of substituted or unsubstituted methionine or any amino acid with similar properties such as a methylated homo-cysteine, lysine, norleucine, leucine or isoleucine;
- X₆ is a residue chosen in the list consisting of substituted or unsubstituted tryptophan, substituted or unsubstituted hetero-tryptophan, substituted or unsubstituted para-tyrosine, substituted or unsubstituted ortho-tyrosine, substituted or unsubstituted meta-tyrosine, substituted or unsubstituted phenylalanine, or substituted or unsubstituted naphthyl-alanine;
- X₁ is the N-terminal side of the molecule of formula (I), X₆ is the C-terminal side of the molecule of formula (I).

Preferably, the hexapeptide sequence of formula (I) comprises at least one substituted or unsubstituted para-tyrosine, substituted or unsubstituted ortho-tyrosine, substituted or unsubstituted meta-tyrosine residue.

According to a specific embodiment, synthetic TSP-1-derived peptide is as described in WO2013/182650, that is to say a peptide comprising the amino acids sequence: KRFYVVMWKK (SEQ ID NO: 1).

In one embodiment, the peptide according to the invention may differ from 1, 2 or 3 amino acids to the SEQ ID NO: 1.

In another embodiment, the peptide according to the invention may differ from 4 or 5 amino acids to the SEQ ID NO: 1.

In one embodiment, the peptide of the invention comprises at least 75% identity over said the SEQ ID NO: 1, even more preferably at least 80%, at least 85%, at least 90%), at least 95%, at least 97% and is still able to induce ICD in tumor cell.

In one embodiment, the peptide of the invention consists in the amino acid sequence as set forth in SEQ ID NO: 1 or a variant thereof comprising at least 75%, preferably at least 80%, 85%, 90%, 95%, 96%, 97%, 98%, 99%, 99.5% or 99.9% identity with SEQ ID NO: 1 and is still able to induce ICD in tumor cells.

In another embodiment of the invention, said peptide is an amino acid sequence of less than 45 amino acids long that comprises the amino acid sequence SEQ ID NO: 1 as defined here above.

In another embodiment of the invention, said peptide is an amino acid sequence of less than 40 amino acids long that comprises the amino acid sequence SEQ ID NO: 1 as defined here above.

In another embodiment of the invention, said peptide is an amino acid sequence of less than 30 amino acids long that comprises the amino acid sequence SEQ ID NO: 1 as defined here above.

In another embodiment of the invention, said peptide is an amino acid sequence of less than 20 amino acids long that comprises the amino acid sequence SEQ ID NO: 1 as defined here above.

In another embodiment of the invention, said peptide is an amino acid sequence of less than 15 amino acids long that comprises the amino acid sequence SEQ ID NO: 1 as defined here above.

According to a specific embodiment, synthetic TSP-1-derived peptide is as described in WO2017/194627, that is to say that it corresponds to a peptide comprising the sequence of formula (II):

-A-B-X₁-X₂-X₃-X₄-X₅-X₆- (II)

wherein
- A and B are amino acid residues, preferably natural or synthetic amino acid residues as defined above;
- X₁, X₂, X₃, X₄, X₅ and X₆ are as defined presently;
or a pharmaceutical salt thereof.

Preferably in formula (II), A is a (D)-Lysine and B is an Arginine.

Preferably in formula (II), A (such as (D)-Lysine) and B (such as (L)-Arginine) are linked to each other by a pseudopeptide bond, such as (-CO-NMe-); more preferably, B is an Arginine and A and B are linked to each other by the bond -CO-NMe-.

Preferably in formula (II), X₁-X₂-X₃-X₄-X₅-X₆ is FYVVXW, FYVVIW, FYVVKW or FYVVLW, wherein X is norleucine.

Alternatively, in formula (II) X₁-X₂-X₃-X₄-X₅-X₆ is FFVVXW, FFVVIW, FFVVKW or FFVVLW, wherein X is norleucine.

In a particular embodiment, the compound of formula (I) is a peptide comprising the sequence of formula (III):

-A-B-X₁-X₂-X₃-X₄-X₅-X₆-C-D- (III)

wherein
- A, B, C and D are amino acid residues, preferably natural or synthetic amino acid residues as defined above;
- X₁, X₂, X₃, X₄, X₅ and X₆ are as defined presently;
or a pharmaceutical salt thereof.

Preferably in formula (III), A is a (D)-Lysine and B is an Arginine; preferably C is an (L)-Lysine and D is a (D)-Lysine.

Preferably in formula (III), A (such as (D)-Lysine) and B (such as (L)-Arginine) are linked to each other by a pseudopeptide bond, such as (-CO-NMe-).

Preferably in formula (III), X₁-X₂-X₃-X₄-X₅-X₆ is FYVVXW, FYVVIW, FYVVKW or FYVVLW, wherein X is norleucine.

Alternatively, in formula (III) X₁-X₂-X₃-X₄-X₅-X₆ is FFVVXW, FFVVIW, FFVVKW or FFVVLW, wherein X is norleucine.

Advantageously, the compound of formula (I) is a peptide comprising the sequence X₁-X₂-X₃-X₄-X₅-X₆ (formula (I)) and to sustain the solubility of the peptide of formula (I), the nature and the size of the structure is comprised between a peptide of 6 and 20 amino acids, more preferably between 7 and 15 amino acids, yet more preferably 8 and 12 amino acids, most preferably 10 amino acids.

More preferably, the compound of formula (I) is a decapeptide (10 amino acids) with a dipeptide linked to the N-terminal extremity of the sequence X₁-X₂-X₃-X₄-X₅-X₆ (formula (I)) via a peptide or pseudopeptide bond, and a dipeptide linked to the C-terminal extremity of the sequence X₁-X₂-X₃-X₄-X₅-X₆ via a peptide or pseudopeptide bond on the N-terminal giving a formula (IV):

Y-A-B-X₁-X₂-X₃-X₄-X₅-X₆-C-D-Z (IV)

wherein
- A, B, C and D are amino acid residues, preferably natural or synthetic amino acid residues as defined above;
- Y is a hydrogen, a C₁-C₆ alkyl group, a C₅-C₈ aryl group, a fragment R₁-CO- wherein R₁ is a hydrogen atom, a C₁-C₆ alkyl group, preferably a methyl, or a C₅-C₈ aryl group, preferably a phenyl;
- Z is a -OH, C₁-C₆ alkyl group, a C₅-C₈ aryl group, a NH₂ group, a C₁-C₆ alkoxy group or a C₅-C₈ aryloxy group;
or a pharmaceutical salt thereof.

Preferably in formula (IV), all the amino residues on either sides of the peptide sequence X₁-X₂-X₃-X₄-X₅-X₆ are natural of (D) or (L) configuration and/or synthetic of (D) or (L) configuration amino acid residues as defined above.

In a specific embodiment, the side chains and/or backbone of the compound of formula (I) are chemically protected according to the above definitions.

Preferably in formula (IV), A is a (D)-Lysine and B is an Arginine.

Preferably C is an (L)-Lysine and D is a (D)-Lysine.

Preferably in formula (IV), A (such as (D)-Lysine) and B (such as (L)-Arginine) are linked to each other by a pseudopeptide bond, such as (-CO-NMe-).

Y is preferably in formula (IV) a hydrogen atom or an acetyl whilst Z is an NH₂.

Preferably in formula (IV), X₁-X₂-X₃-X₄-X₅-X₆ is FYVVXW, FYVVIW, FYVVKW or FYVVLW, wherein X is norleucine.

Alternatively in formula (IV), X₁-X₂-X₃-X₄-X₅-X₆ is FFVVXW, FFVVIW, FFVVKW or FFVVLW, wherein X is norleucine.

The present invention concerns a compound or the pharmaceutical acceptable salt thereof as presently disclosed, characterized in that X₁, X₂, X₅ and/or X₆ are non-ionic charged amino acid residues, such as X₅ is a norleucine, leucine or isoleucine residue, preferably a norleucine residue.

The present invention concerns a compound or the pharmaceutical acceptable salt thereof as presently disclosed, characterized in that the at least one pseudopeptide bond is an N-methyl peptide bond, preferably in a fragment linked to X₁ on the N-terminal side of the compound of formula (I) and/or in a fragment linked to X₆ on the C-terminal side of the compound of formula (I).

The present invention concerns a compound or the pharmaceutical acceptable salt thereof as presently disclosed, characterized in that a hydrogen atom, an amino acid residue or a peptide fragment is linked on the N-terminal amine of hexapeptide of formula (I).

The present invention concerns a compound or the pharmaceutical acceptable salt thereof as presently disclosed, characterized in that an -OH group, a -NH₂ group, an amino acid residue or a peptide fragment is linked to the C-terminal carbonyl of the hexapeptide of formula (I).

The present invention concerns a compound or the pharmaceutical acceptable salt thereof as presently disclosed, characterized in that the N-terminal amine of compound (I) or a pharmaceutical salt thereof is capped by a non-ionic charged group preferably chosen from the list consisting of a C₁-C₆ alkyl group, a C₅-C₈ aryl group, a fragment R₁-CO- wherein R₁ is:
- a hydrogen atom,
- a C₁-C₆ alkyl group, preferably a methyl or
- a C₅-C₈ aryl group, preferably a phenyl.

The present invention concerns a compound or the pharmaceutical acceptable salt thereof as presently disclosed, characterized in that the C-terminal carboxylic acid has been replaced by a non-ionic charged group such as COR₂ wherein R₂ is a C₁-C₆ alkyl group, a C₅-C₈ aryl group, a NH₂ group, a C₁-C₆ alkoxy group or a C₅-C₈ aryloxy group.

The present invention concerns a compound or the pharmaceutical acceptable salt thereof as presently disclosed, characterized in that said compound or the pharmaceutical acceptable salt thereof comprises the sequence YVV, preferably in position X₂-X₃-X₄.

The present invention concerns a compound or the pharmaceutical acceptable salt thereof as presently disclosed, characterized in that X₅ is a lysine, norleucine, leucine or isoleucine.

The present invention concerns a compound or the pharmaceutical acceptable salt thereof as presently disclosed, characterized in that X₅ is a lysine, norleucine, leucine or isoleucine and X₁ is a phenylalanine.

The present invention concerns a compound or the pharmaceutical acceptable salt thereof as presently disclosed, characterized in that X₅ is a lysine, norleucine, leucine or isoleucine and X₂ is a tyrosine. The present invention concerns a compound or the pharmaceutical acceptable salt thereof as presently disclosed, characterized in that X₅ is a lysine, norleucine, leucine or isoleucine and X₂ is a phenylalanine.

The present invention concerns a compound or the pharmaceutical acceptable salt thereof as presently disclosed, characterized in that X₅ is a lysine, norleucine, leucine or isoleucine and X₃ is a valine.

The present invention concerns a compound or the pharmaceutical acceptable salt thereof as presently disclosed, characterized in that X₅ is a lysine, norleucine, leucine or isoleucine and X₄ is a valine.

The present invention concerns a compound or the pharmaceutical acceptable salt thereof as presently disclosed, characterized in that X₅ is a lysine, norleucine, leucine or isoleucine and X₆ is a tryptophan.

The present invention concerns a compound or the pharmaceutical acceptable salt thereof as presently disclosed, characterized in that X₅ is a lysine, norleucine, leucine or isoleucine, X₁ is a phenylalanine and X₂ is a tyrosine. The present invention concerns a compound or the pharmaceutical acceptable salt thereof as presently disclosed, characterized in that X₅ is a lysine, norleucine, leucine or isoleucine, X₁ is a phenylalanine and X₂ is a phenylalanine.

The present invention concerns a compound or the pharmaceutical acceptable salt thereof as presently disclosed, characterized in that X₅ is a lysine, norleucine, leucine or isoleucine, X₁ is a phenylalanine and X₃ is a valine.

The present invention concerns a compound or the pharmaceutical acceptable salt thereof as presently disclosed, characterized in that X₅ is a lysine, norleucine, leucine or isoleucine, X₁ is a phenylalanine and X₄ is a valine.

The present invention concerns a compound or the pharmaceutical acceptable salt thereof as presently disclosed, characterized in that X₅ is a lysine, norleucine, leucine or isoleucine, X₁ is a phenylalanine and X₆ is a tryptophan.

The present invention concerns a compound or the pharmaceutical acceptable salt thereof as presently disclosed, characterized in that X₅ is a lysine, norleucine, leucine or isoleucine, X₁ is a phenylalanine, X₂ is a tyrosine and X₃ is a valine. The present invention concerns a compound or the pharmaceutical acceptable salt thereof as presently disclosed, characterized in that X₅ is a lysine, norleucine, leucine or isoleucine, X₁ is a phenylalanine, X₂ is a phenylalanine and X₃ is a valine.

The present invention concerns a compound or the pharmaceutical acceptable salt thereof as presently disclosed, characterized in that X₅ is a lysine, norleucine, leucine or isoleucine, X₁ is a phenylalanine, X₂ is a tyrosine and X₄ is a valine. The present invention concerns a compound or the pharmaceutical acceptable salt thereof as presently disclosed, characterized in that X₅ is a lysine, norleucine, leucine or isoleucine, X₁ is a phenylalanine, X₂ is a phenylalanine and X₄ is a valine.

The present invention concerns a compound or the pharmaceutical acceptable salt thereof as presently disclosed, characterized in that X₅ is a lysine, norleucine, leucine or isoleucine, X₁ is a phenylalanine, X₂ is a tyrosine and X₆ is a tryptophan. The present invention concerns a compound or the pharmaceutical acceptable salt thereof as presently disclosed, characterized in that X₅ is a lysine, norleucine, leucine or isoleucine, X₁ is a phenylalanine, X₂ is a phenylalanine and X₆ is a tryptophan.

The present invention concerns a compound or the pharmaceutical acceptable salt thereof as presently disclosed, characterized in that X₅ is a lysine, norleucine, leucine or isoleucine, X₁ is a phenylalanine, X₂ is a tyrosine, X₃ is a valine and X₄ is a valine. The present invention concerns a compound or the pharmaceutical acceptable salt thereof as presently disclosed, characterized in that X₅ is a lysine, norleucine, leucine or isoleucine, X₁ is a phenylalanine, X₂ is a phenylalanine, X₃ is a valine and X₄ is a valine.

The present invention concerns a compound or the pharmaceutical acceptable salt thereof as presently disclosed, characterized in that X₅ is a lysine, norleucine, leucine or isoleucine, X₁ is a phenylalanine, X₂ is a tyrosine, X₃ is a valine and X₆ is a tryptophan. The present invention concerns a compound or the pharmaceutical acceptable salt thereof as presently disclosed, characterized in that X₅ is a lysine, norleucine, leucine or isoleucine, X₁ is a phenylalanine, X₂ is a phenylalanine, X₃ is a valine and X₆ is a tryptophan.

The present invention concerns a compound or the pharmaceutical acceptable salt thereof as presently disclosed, characterized in that X₅ is a lysine, norleucine, leucine or isoleucine, X₁ is a phenylalanine, X₂ is a tyrosine, X₃ is a valine X₄ is a valine and X₆ is a tryptophan. The present invention concerns a compound or the pharmaceutical acceptable salt thereof as presently disclosed, characterized in that X₅ is a lysine, norleucine, leucine or isoleucine, X₁ is a phenylalanine, X₂ is a phenylalanine, X₃ is a valine X₄ is a valine and X₆ is a tryptophan.

The present invention concerns a compound or the pharmaceutical acceptable salt thereof as presently disclosed, characterized in that said compound or the pharmaceutical acceptable salt thereof comprises the sequence YVV-norleucine (SEQ ID 5), preferably in position X₂-X₃-X₄-X₅.

The present invention concerns a compound or the pharmaceutical acceptable salt thereof as presently disclosed, characterized in that X₁, X₂, and/or X₆ is a para-fluoro-phenylalanine, para-amino-phenylalanine or para-nitro-phenylalanine.

The present invention concerns a compound or the pharmaceutical acceptable salt thereof as presently disclosed, characterized in that X₁ is a substitutes or unsubstituted phenylalanine, X₂ is a substituted or unsubstituted paratyrosine, and X₆ is a substituted or unsubstituted tryptophane.

The present invention concerns a compound or the pharmaceutical acceptable salt thereof as presently disclosed, characterized in that X₁ is a substitutes or unsubstituted phenylalanine, X₂ is a substituted or unsubstituted phenylalanine, and X₆ is a substituted or unsubstituted tryptophane.

The present invention concerns a compound or the pharmaceutical acceptable salt thereof as presently disclosed, characterized in that X₁ is a substitutes or unsubstituted phenylalanine, X₂ is a unsubstituted phenylalanine, and X₆ is a substituted or unsubstituted tryptophane.

The present invention concerns a compound or the pharmaceutical acceptable salt thereof as presently disclosed, characterized in that the hexapeptide of formula (I) is comprised between two amino acid residues of the (D) configuration, such as two (D)-lysines.

Example of peptides of the invention are listed below:
KRFYVVMWKK (4N1K, SEQ. ID. N°1, cf. chemical structure below)
(D)K-R-F-Y-V-V-M-K-(D)K (PKHB1, SEQ. ID. N°2, cf. chemical structure below)
H-(D)K ψ(CONMe)R F Y V V X W K (D)K-OH (PKT16, SEQ. ID. N°17, cf. chemical structure below)

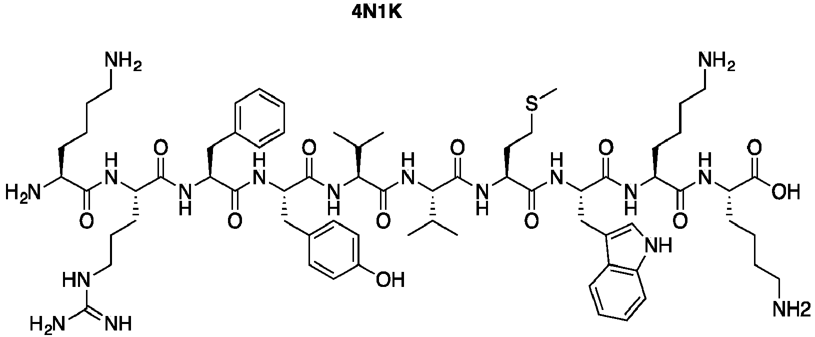

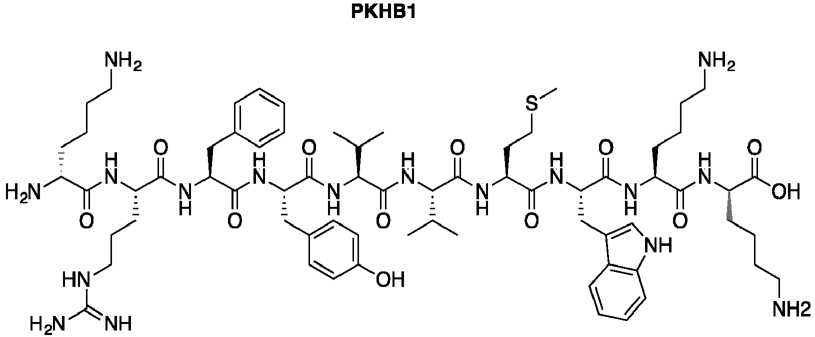

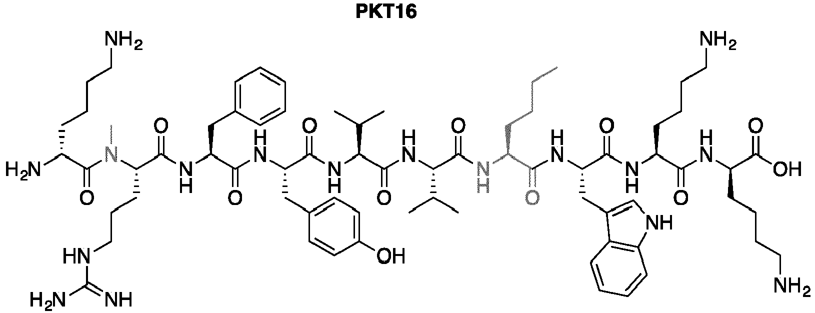

Other examples of compounds or of pharmaceutical acceptable salt thereof are:

| | |
|---|---|
| Ac-RFYVVMWK-NH₂ | (SEQ. ID. N°3) |
| Ac-KRFYVVMWKK-NH₂ | (SEQ. ID. N°4) |
| H-(D)KAFYVVMWK(D)K-OH | (SEQ. ID. N°5) |
| H-(D)KRFYVV(Nle)WK(D)K-OH | (SEQ. ID. N°6) |
| H-FYVVXW-OH | (SEQ. ID. N°7) |
| H-FYVVXW-NH₂ | (SEQ. ID. N°8) |
| Ac-FYVVXW-OH | (SEQ. ID. N°9) |
| Ac-FYVVXW-NH₂ | (SEQ. ID. N°10) |
| H-(D)KFYVVXW(D)K-OH | (SEQ. ID. N°11) |
| H-FYVVKW-OH | (SEQ. ID. N°12) |
| H-FYVVKW-NH₂ | (SEQ. ID. N°13) |
| H-(D)K ψ(CONMe)RFYVVMWK(D)K-OH | (SEQ. ID. N°14) |
| H-(D)KRFYVVMWψ(CONMe)K (D)K-OH | (SEQ. ID. N°15) |
| H-(D)Kψ(CONMe)RFYVVMWψ(CONMe)K | (D)K-OH (SEQ. ID. N°16) |
| H-(D)K ψ(CONMe)RFYVVLWK(D)K-OH | (SEQ. ID. N°18) |
| H-(D)K ψ(CONMe)RFYVVIWK(D)K-OH | (SEQ. ID. N°19) |
| H-(D)K ψ(CONMe)RFFVVXWK(D)K-OH | (SEQ. ID. N°20) |

in the above peptides:
- the "H" on the left hand side of the structures represents a hydrogen atom,
- the term "Ac" means that the N-terminal amine is acetylated,
- the "OH" on the right hand side of the structures represents the OH of the C-terminal COOH,
- the "X" represents norleucine residue,
- the "NH₂" on the right hand side of the structures means that the OH of the C-terminal COOH has been replaced by NH₂,
- the (D) means that the following amino acid residue is of the (D) configuration, the terms "*h*R" and "*h*K" represent homo-arginine and homo-lysine respectfully,
- "ψ(CONMe)" represent the pseudopeptide bond linking the two amino acid residues on either side of this term, and
- Nle represents a norleucine residue.

According to another embodiment, synthetic TSP-1-derived peptides according to the invention are isolated cyclic peptide of general formula (V) as described in WO 2017/194634: 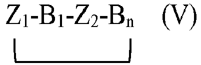 or a pharmacologically acceptable salt or a biologically active derivative thereof, wherein:
- **Z₁** is nothing or an heterochiral sequence D-Pro-L-Pro (also designated p-P, p being a D-proline and P a L-proline) or any sequence of two amino acids or analogs of amino acid able to mimic said heterochiral sequence or mimic a beta turn, example of amino acids or analogs of amino acid of said sequence are nipecotic acid, isonipecotic acid, piperidine carboxylic acid, silaproline, thioproline and any other substituted derivative thereof (fluoro, methyl, bromo etc), pseudo proline, substituted proline, N-methyl amino acids, cyclopropyl amino acids (see Karoyan et al. Target in heterocyclic system, 2004 and Karoyan et al. ChemBioChem (2011), 12(7), 1039-1042 and Larregola et al. Journal of Peptide Science (2011), 17(9), 632-643) or biaryl amino acids templates; in a preferred embodiment, Z₁ is D-Pro-L-Pro;
- **B₁** represents the peptidic sequence X₇-X₈-X₉-X₁₀-X₁₁-X₁₂-X₁₃-X₁₄-X₁₅-X₁₆ derived from the beta-strand N°7 of TSP-1 (of sequence RFYVVMWK) wherein:
   **X₇** refers to nothing or serine or any amino acid with similar properties such as glycine or alanine or threonine;
   **X₈** refers to nothing or arginine or any amino acid with similar properties such as homoarginine, lysine, ornithine, phenylalanine, naphtylalanine, N-methyl arginine or homophenylalanine or any other ring substituted analogues in ortho, meta or para position; for example for arginine, derivatives include any other side chain involving a guanido function and/or one or more than one amine function;
   **X₉** refers to phenylalanine or any amino acid with similar properties including naphtylalanine, homophenylalanine or any other ring substituted analogues in ortho, meta or para position such as para-fluoro-phenylalanine, para-amino-phenylalanine or para-nitro-phenylalanine; tyrosine or any amino acid with aromatic side chains;
   **X₁₀** refers to tyrosine or any amino acid with aromatic side chains, phenylalanine or any amino acid with similar properties including naphtylalanine, homophenylalanine or any other ring substituted analogues in ortho, meta or para position such as para-fluoro-phenylalanine, para-amino-phenylalanine or para-nitro-phenylalanine;
   **X₁₁** refers to valine or any amino acid with similar properties including leucine, isoleucine, terleucine, methionine;
   **X₁₂** refers to valine or any amino acid with similar properties including leucine, isoleucine, terleucine, methionine;
   **X₁₃** refers to methionine or lysine or any amino acid with similar properties including valine, methionine, norleucine, leucine or isoleucine or terleucine;
   **X₁₄** refers to tryptophan, tyrosine, phenylalanine, naphthyl-alanine, para-fluoro-phenylalanine, para-amino-phenylalanine, para-nitro-phenylalanine, D-prolino-tryptophane or D-prolino-homotryptophane;
   **X₁₅** refers to nothing or lysine or any amino acid with similar properties including arginine, homoarginine, ornithine, phenylalanine, naphtylalanine, N-methyl arginine or homophenylalanine or any other ring substituted analogues in ortho, meta or para position or histidine;
   **X₁₆** refers to nothing or glutamine or alanine or any amino acid with similar properties including asparagine;
   preferably, if Xg is nothing then X₇ is nothing and/or if X₁₅ is nothing then X₁₆ is nothing; preferably, B₁ comprises at least the 6 amino acids -X₉-X₁₀-X₁₁-X₁₂-X₁₃-X₁₄-; more preferably, B₁ comprises at least the peptidic fragment -FYVVMW-;
- **Z₂** is nothing or an heterochiral sequence D-Pro-L-Pro (also designated p-P) or any sequence of two amino acids or analogs of amino acid able to mimic said heterochiral sequence or mimic a beta turn, example of amino acids or analogs of amino acid of said sequence are nipecotic acid, isonipecotic acid, piperidine carboxylic acid, silaproline, thioproline and any other substituted derivative thereof (fluoro, methyl, bromo etc), pseudo proline, substituted proline, N-methyl amino acids, cyclopropyl amino acids (Karoyan et al. Target in heterocyclic system, 2004 and Karoyan et al. ChemBioChem (2011), 12(7), 1039-1042 and Larregola et al. Journal of Peptide Science (2011), 17(9), 632-643) or biaryl amino acids templates; in a preferred embodiment, Z₂ is D-Pro-L-Pro;
- **Bₙ** represents B₁ or B₂;
- **B₂** is nothing or a peptidic sequence comprising between 6 and 10 amino acids derived from the beta-strand N°8 of TSP-1 (of sequence GLSVKVVNS) ; in an embodiment, B₂ comprises the following sequence: -X₂₂-X₁₇-X₁₈-X₂₃-X₂₄-X₁₉-X₂₅-X₂₆-X₂₀-X₂₁-; preferably, B₂ comprises the following sequence -X₂₂-X₁₇-X₁₈-S-V-X₁₉-V-V-X₂₀-X₂₁- wherein:
   **X₁₇** is nothing or glycine or alanine or any amino acid with similar properties including serine;
   **X₁₈** is isoleucine or leucine or alanine or any amino acid with similar properties including terleucine, valine, methionine;
   **X₁₉** is lysine or alanine or any amino acid with similar properties including arginine, homoarginine, lysine, ornithine, phenylalanine, naphtylalanine, N-methyl arginine or homophenylalanine or any other ring substituted analogues (ortho, meta, para), histidine, or methionine or any amino acid with similar properties including valine, leucine, isoleucine, terleucine;
   **X₂₀** is nothing or asparagine or alanine or any amino acid with similar properties including glutamine or lysine or any amino acid with similar properties including arginine, homoarginine, lysine, ornithine, phenylalanine, naphtylalanine, N-methyl arginine or homophenylalanine or any other ring substituted analogues (ortho, meta, para), histidine;
   **X₂₁** is nothing, serine or glycine or any amino acid with similar properties;
   **X₂₂** is nothing or serine or alanine or any amino acid with similar properties;
   **X₂₃** is serine or alanine or any amino acid with similar properties including leucine, isoleucine, terleucine;
   **X₂₄** is valine or alanine or any amino acid with similar properties including leucine, isoleucine, terleucine;
   **X₂₅** is valine or alanine or any amino acid with similar properties including leucine, isoleucine, terleucine; and
   **X₂₆** is valine or alanine or any amino acid with similar properties including leucine, isoleucine, terleucine;
   preferably, if X₁₇ is nothing then X₂₂ is nothing and/or if X₂₀ is nothing then X₂₁ is nothing; preferably B₂ comprises at least the 6 amino acids -X₁₈-S-V-X₁₉-V-V-; more preferably, B₂ comprises at least the peptidic fragment -LSVKVV-;
and wherein said isolated cyclic peptide comprises between 8 and 26 amino acids, preferably between 14 and 22 amino acids; according to an other embodiment, isolated cyclic peptide comprises between 18 and 22 amino acids.

The isolated cyclic peptide of general formula (V) of the invention yet comprises at least parts of the beta-sheet N°7 or of the beta-sheets N°7 and 8 of the C-terminal domain of the TSP-1 but cannot be the entire sequence of the C-terminal domain of the TSP-1 (as described by Kosfeld MD, Frazier WA (1993) Identification of a new cell adhesion motif in two homologous peptides from the COOH-terminal cell binding domain of human thrombospondin. J Biol Chem 268: 8806-8814), because this domain has not the same biologic activity as cyclic peptides of the invention.

Except when explicitly mentioned, all amino acids are indifferently of the (D) or (L) configuration.

The present invention thus encompasses cyclic peptides of formula B₁-B₂, Z₁-B₁-B₂, B₁-Z₂-B₁, B₁-B₁ (each B₁ being identical or different) and B₁-Z₂-B₁ (each B₁ being identical or different).

Preferably, isolated cyclic peptide comprises an even number of amino acids (that is to say B₁ and Bₙ have the same number of amino acids and both consist in a fragment of 6, 7, 8, 9 or 10 amino acids) and wherein said isolated cyclic peptide comprises between 8 and 26 amino acids, preferably between 12 and 22 amino acids; more preferably, isolated cyclic peptides of the invention consist in 12, 14, 16, 18, 20 or 22 amino acids.

In a preferred embodiment, B₁ and B₂ are arranged so that X₅ of B₁ faces X₁₆ of B₂ and X₈ of B₁ faces X₁₅ of B₂ as illustrated below:

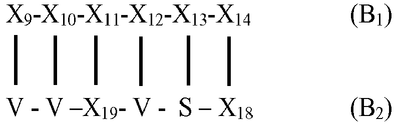

According to a particular embodiment, both Z₁ and Z₂ can be nothing; if Z₁ consists in two amino acids then Z₂ is nothing and if Z₂ consists in two amino acids then Z₁ is nothing.

Synthesis of said cyclic peptides is described on WO 2017/194634.

Examples of isolated cyclic peptide according to the present invention are as described in Table I:

**Table I**

| **PEPTIDES** | **STRUCTURE / AMINO ACID SEQUENCE (linear representation)** | **FORMULA AND MW** |
|---|---|---|
| PKD10 (SEQ. ID. N°21) | 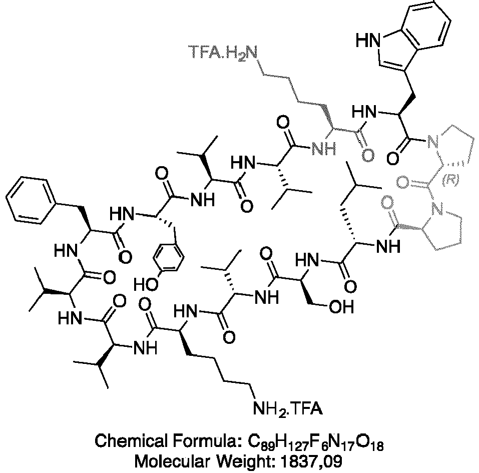 | |
| | -F-Y-V-V-K-W-p-P-L-S-V-K-V-V- | |
| PKDIOFF (SEQ. ID. N°22) | 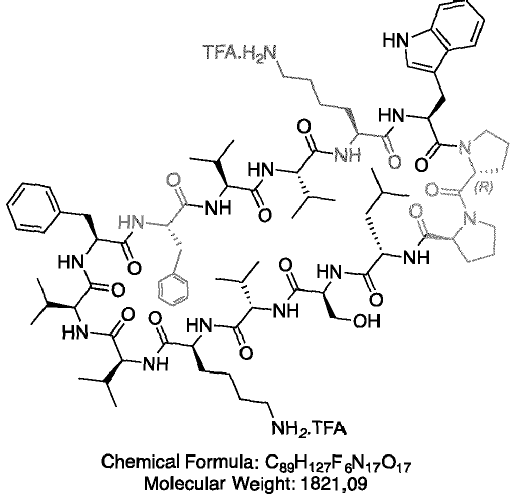 | |
| | -F-F-V-V-K-W-p-P-L-S-V-K-V-V- | |
| PKTDi2 (SEQ. ID. N°23) | 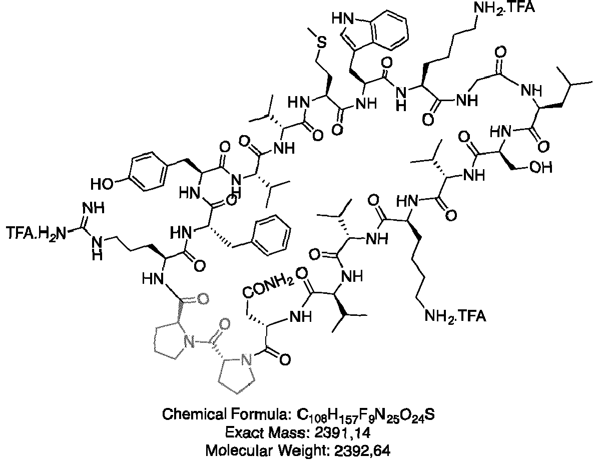 | |
| | -p-P-R-F-Y-V-V-M-W-K-G-L-S-V-K-V-V-N- | |
| PKTD1 (SEQ. ID. N°24) | 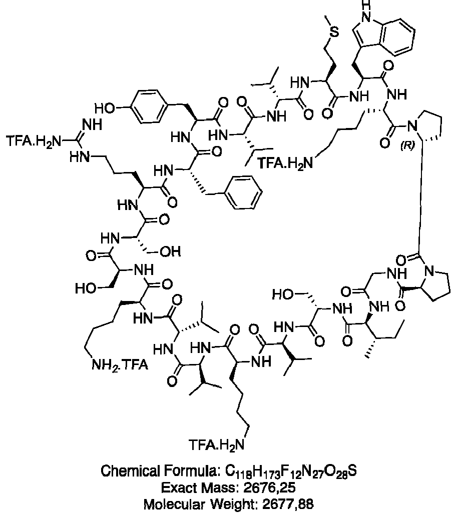 | |
| | -S-R-F-Y-V-V-M-W-K-p-P-G-I-S-V-K-V-V-K-S- | |
| PKTD10 (SEQ. ID. N°25) | 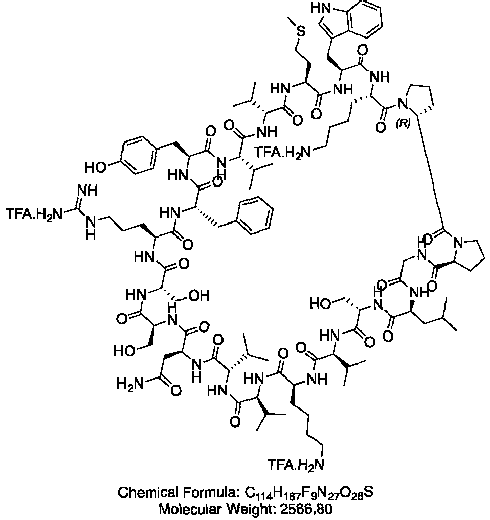 | |
| | -S-R-F-Y-V-V-M-W-K-p-P-G-L-S-V-K-V-V-N-S- | |
| PKTD10-RNMe (SEQ. ID. N°26) | 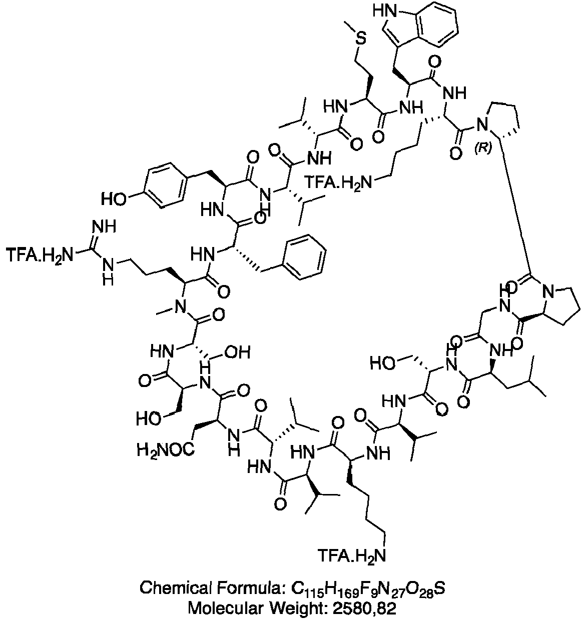 | |
| | -S-R*-F-Y-V-V-M-W-K-p-P-G-L-S-V-K-V-V-N-S- R* = RNMe | |
| PKTD10-X-RNMe (SEQ. ID. N°27) | 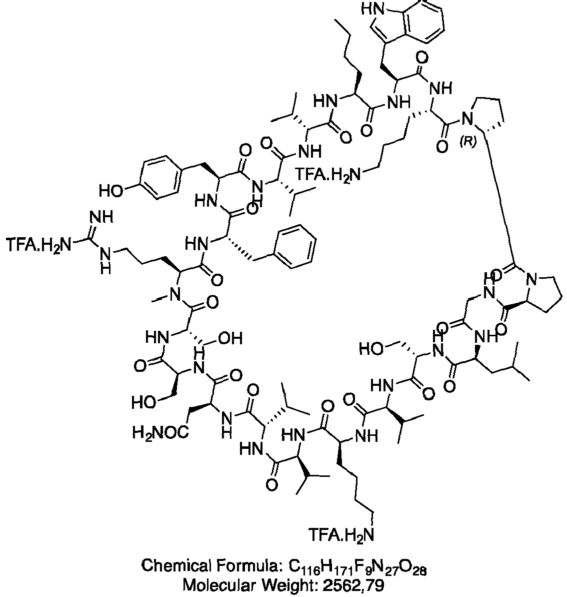 | |
| | -S-R*-F-Y-V-V-X-W-K-p-P-G-L-S-V-K-V-V-N-S- R* = RNMe and X = NLe | |
| PKTD10-3-X-RNMe (SEQ. ID. N°28) | 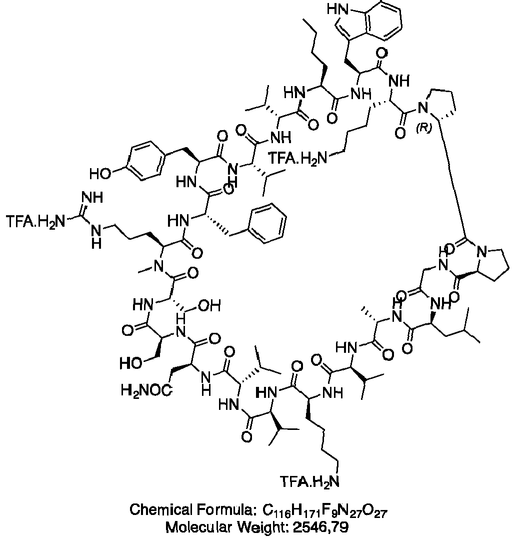 | |
| | -S-R*-F-Y-V-V-X-W-K-p-P-G-L-A-V-K-V-V-N-S- R* = RNMe and X =N Le | |
| PKTD16 (SEQ. ID. N°29) | -S-R-F-Y-V-V-M-W-K-p-P-S-R-F-Y-V-V-M-W-K- | |
| PKTD18 (SEQ. ID. N°30) | 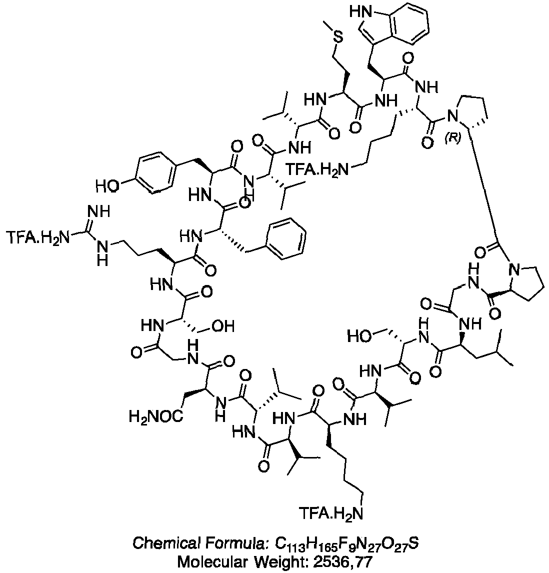 | |
| | -S-R-F-Y-V-V-M-W-K-p-P-G-L-S-V-K-V-V-N-G- | |
| PKTD11 (SEQ. ID. N°31) | 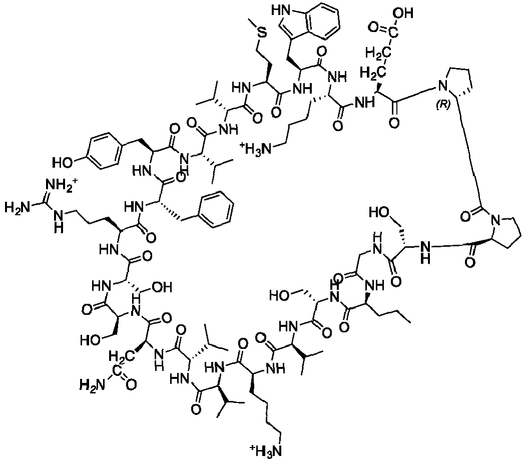 | Chemical Formula: C₁₁₅H₁₈₀N₂₉O₃₀S₃₄ |
| | | Molecular Weight: 2480,94 |
| | -S-R-F-Y-V-V-M-W-K-Q-p-P-S-G-L-S-V-K-V-V-N-S- | |
| PKTD11-RNMe (SEQ. ID. N°32) | 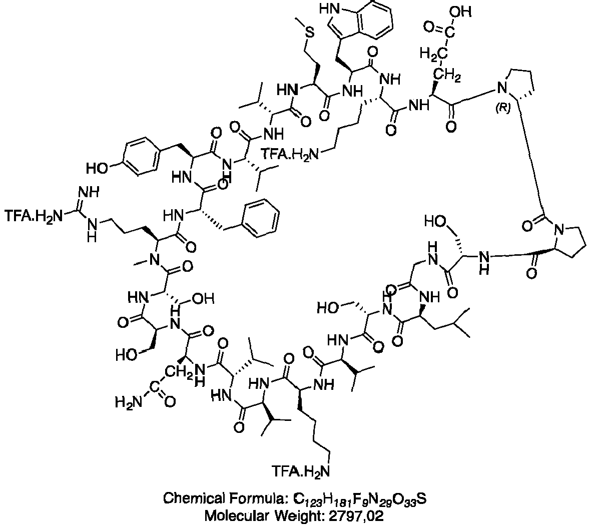 | |
| | -S-R*-F-Y-V-V-M-W-K-Q-p-P-S-G-L-S-V-K-V-V-N-S- R* = RNMe | |
| PKTD11-X-RNMe (SEQ. ID. N°33) | 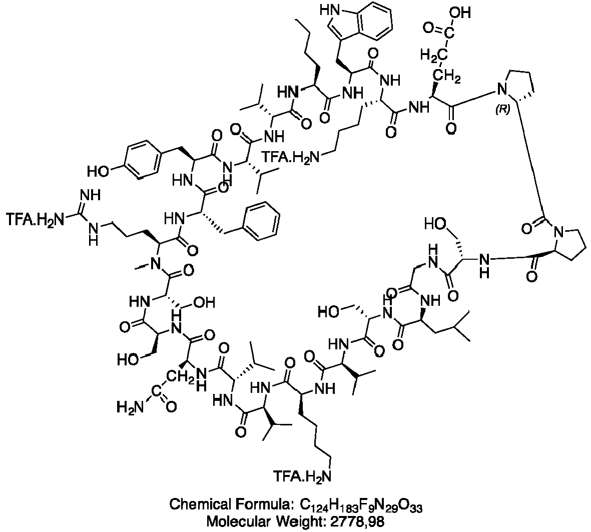 | |
| | -S-R*-F-Y-V-V-X-W-K-Q-p-P-S-G-L-S-V-K-V-V-N-S- R* = RNMe and X = NLe | |

The present invention also relates to the use of variants of the above-defined peptides; said variants include protein having amino acid alterations such as deletions, insertions and/or substitutions.

A "deletion" refers to the absence of one or more amino acids in the protein.

An "insertion" refers to the addition of one or more of amino acids in the protein.

A "substitution" refers to the replacement of one or more amino acids by another amino acid residue in the protein.

Typically, a given amino acid is replaced by an amino acid having similar properties (such as, for example, polarity, hydrogen bonding potential, acidic, basic, hydrophobic, aromatic, and the like). This given amino acid can be a natural amino acid or a non natural amino acid. Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm.

Typically, the invention encompasses peptides substantially identical to the above-defined peptides in which one or more residues have been conservatively substituted with a functionally similar residue and which displays the functional aspects of the synthetic TSP-1 derived peptides as described here above, i.e. being still able to induce ICD in substantially the same way as a peptide consisting of the given amino acid sequence.

Examples of conservative substitutions include the substitution of one non-polar (hydrophobic) residue such as isoleucine, valine, leucine or methionine for another, the substitution of one polar (hydrophilic) residue for another such as between arginine and lysine, between glutamine and asparagine, between glycine and serine, the substitution of one basic residue such as lysine, arginine or histidine for another, or the substitution of one acidic residue, such as aspartic acid or glutamic acid or another.

The term "conservative substitution" also includes the use of a chemically derivatized residue in place of a non-derivatized residue.

"Chemical derivative" refers to a subject peptide having one or more residues chemically derivatized by reaction of a functional side group.

Examples of such derivatized molecules include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im- benzylhistidine. Chemical derivatives also include peptides that contain one or more naturally-occurring amino acid derivatives of the twenty standard amino acids. For examples: 4-hydroxyproline may be substituted for proline; 5-hydroxylysine may be substituted for lysine; 3-methylhistidine may be substituted for histidine; homoserine may be substituted for serine; and ornithine may be substituted for lysine. The term "conservative substitution" also includes the use of non natural amino acids aimed to control and stabilize peptides or proteins secondary structures. These non natural amino acids are chemically modified amino acids such as prolinoamino acids, beta-amino acids, N-methylamino acids, cyclopropylamino acids, alpha,alpha-substituted amino acids as describe here below. These non natural amino acids may include also fluorinated, chlorinated, brominated- or iodinated modified amino acids.

Preferably, the synthetic TSP-1-derived peptide are selected amongst PKHB1 (SEQ. ID. N°2), PKT16 (SEQ. ID. N°17) and PKD10 (SEQ. ID. N°43).

The synthetic TSP-1-derived peptide are used to induce immunogenic cancer cell death for treating any cancers or neoplasia; for example, cancer is selected form the group consisting of adrenal cortical cancer, anal cancer, bile duct cancer, multiple myeloma, bladder cancer, bone cancer, brain and central nervous system cancer, breast cancer, Castleman disease, cervical cancer, colorectal cancer, endometrial cancer, esophagus cancer, gallbladder cancer, gastrointestinal carcinoid tumors, Hodgkin's disease, non-Hodgkin's lymphoma, Kaposi's sarcoma, kidney cancer, laryngeal and hypopharyngeal cancer, leukemia, liver cancer, lung cancer, mesothelioma, plasmacytoma, nasal cavity and paranasal sinus cancer, nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma, salivary gland cancer, skin cancer, melanoma and metastatic melanoma, stomach cancer, testicular cancer, thymus cancer, thyroid cancer, vaginal cancer, vulvar cancer, and uterine cancer, preferably leukemia like chronic lymphocytic leukemia, acute lymphoblastic leukemia.

According to another embodiment, the present invention relates to the *in vitro* use of synthetic TSP-1-derived peptide for inducing immunogenic cell death in tumour cell.

The tumour cell mentioned in the present invention is a cell obtained from a tumor of a subject suffering from a cancer, in particular from at least one of the previously identified cancers. It is to be understood that the expression "tumour cells" used to identify cells obtained from a tumor of a subject, is also used, in the present description, to identify circulating tumor cells (in the case of leukaemia for example), cells obtained from a tumor bed, or cells obtained from a metastase.

The present invention also relates to a tumour cell treated with a synthetic TSP-1-derived peptide, also designated by "vaccine used for immunostimulation", for its use to induce immunogenic cell death for the treatment of cancer.

The vaccine used for immunostimulation is obtained by culturing said tumour cell and then treating the obtained culture of tumour cells with a synthetic TSP-1-derived peptide.

An example of such a treatment is described in the experimental part.

Preferably the "tumour cell treated with a synthetic TSP-1-derived peptide" consists in death tumour cells.

The present invention also relates to the use of a synthetic TSP-1-derived peptide for the preparation of tumour cell usable according to the present invention and to process for preparation of such tumour cell comprising a step of treating said cells with a synthetic TSP-1-derived peptide.

The present invention further relates to pharmaceutical composition, preferably injectable, comprising a tumour cell treated with a synthetic TSP-1-derived peptide and a pharmaceutically acceptable carrier.

In a particular embodiment, the pharmaceutical preparation that will be injected is obtained by treatment of the tumor (solid or liquid) with synthetic TSP-1-derived peptide, after collecting blood sample from patients for liquid tumors (leukemia) or after surgery for solid tumors to obtained at least 10⁶ cells that will be cultured at then treated with synthetic TSP-1-derived peptide. The administration (injection) of the pharmaceutical preparation may occur before, simultaneously and/or after a "conventional treatment of cancer" that may be selected from a chemotherapy, a radiotherapy, an hormonotherapy, an immunotherapy, a specific kinase inhibitor-based therapy, an antiangiogenic agent based-therapy, an antibody-based therapy, in particular a monoclonal antibody-based therapy, for liquid tumors and after surgery for the solid tumor.

For the purpose of the invention, suitable pharmaceutically acceptable carriers include, but are not limited to: water, salt solutions (e.g., NaCI), alcohols, gum arabic, vegetable oils, benzyl alcohols, polyethylene glycols, gelatin, carbohydrates such as lactose, amylose or starch, magnesium stearate, talc, silicic acid, viscous paraffin, perfume oil, fatty acid esters, hydroxymethylcellulose, and polyvinyl pyrolidone, lipids such as but not limited to :phospholipids, sphinglipids, glycerol-fatty acid esters...

The pharmaceutical composition of the invention can be sterilized and if desired, mixed with auxiliary agents, e. g., lubricants, preservatives, stabilizers, wetting agents, emulsifiers, salts for influencing osmotic pressure, buffers, coloring, flavoring and/or aromatic substances and the like which do not deleteriously react with the active compounds. The pharmaceutical composition of the invention, if desired, can also contain minor amounts of wetting or emulsifying agents, or pH buffering agents.

The pharmaceutical composition of the invention can be a liquid solution, suspension, emulsion,. Some appropriate precise formulations are described, for example, in Remington, The Science and Practice of Pharmacy, 19th edition, 1995, Mack Publishing Company.

The pharmaceutical composition of the invention can be formulated in accordance with the routine procedures as a composition adapted for intravenous administration to an individual. Typically, compositions for intravenous administration are solutions in sterile isotonic aqueous buffer or a sterile lyophilized formulation to be reconstituted prior injection, such injection can be intravenous, intramuscular, subcutaneous, intrathecal, such pharmaceutical composition can also be inhaled through nasal and/or pulmonary delivery. In a preferred embodiment the pharmaceutical composition of the invention is a liquid composition that is dedicated to be administered by injection, and for example, by intratumoral injection. Said intratumoral injection can be obtained for example by using stereotactic neurosurgery. This administration can be performed prior to or after a surgical operation intended to remove the tumor. In the first case, the composition enables to inhibit the growth of the tumor and avoid dissemination of the tumor cells and the occurrence of dramatic symptoms on the subject; in the second case, the composition can be used to destroy all the tumor cells that have not be removed during the surgical operation.

The effective dose of the synthetic TSP-1-derived peptide or of the "tumour cell treated with a synthetic TSP-1-derived peptide" varies in function of numerous parameters such as, for example, the chosen administration method, the weight, age, sex, and the sensitivity of the individual to be treated. Consequently, the optimal dose must be determined individually, in function of the relevant parameters, by a medical specialist. In order to predict the expected active doses in human from the first animal studies presented hereunder, one can also use the fc₂ and C_{T} values as described by Rocchetti et al (2007).

According to another embodiment, the present invention relates to a method of treatment of cancer comprising a step of administration to a subject in need thereof a synthetic TSP-1-derived peptide and/or tumour cell treated with a synthetic TSP-1-derived peptide to a patient in a amount sufficient to induce ICD.

### FIGURES LEGENDS

**Figure 1****.** CTD of TSP-1 from pdb 1ux6. **A.** The T3₅₋₇-CTD C974S/N1049K double mutant crystallised in the presence of 5 mM calcium (resolution 1.9 Å) from Ala813 to Pro1151 composed of 15 β-strands. **B.** Representation of 10 β-strands from the lectin-like
β-sandwich. The β-strands 7 and 8 have been coloured respectively in orange (strand 7, RFYVVMWK) and blue (strand 8, GLSKVVK) highlighting the antiparallel β-sheets formed by association of strands [7,8].

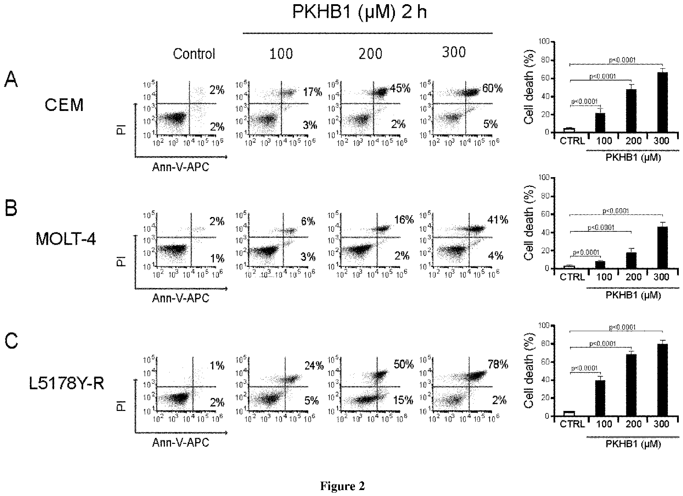

**Figure 2****. PKHB1 induces cell death in T-ALL leukemia cell lines.** Cell death was measured by Annexin-V-APC and PI staining and graphed **A.** CEM, **B.** MOLT-4 human leukemia cells, and C. L5178Y murine cell line, without treatment (Control) and treated with 100, 200 and 300µM PKHB1 for 2h.

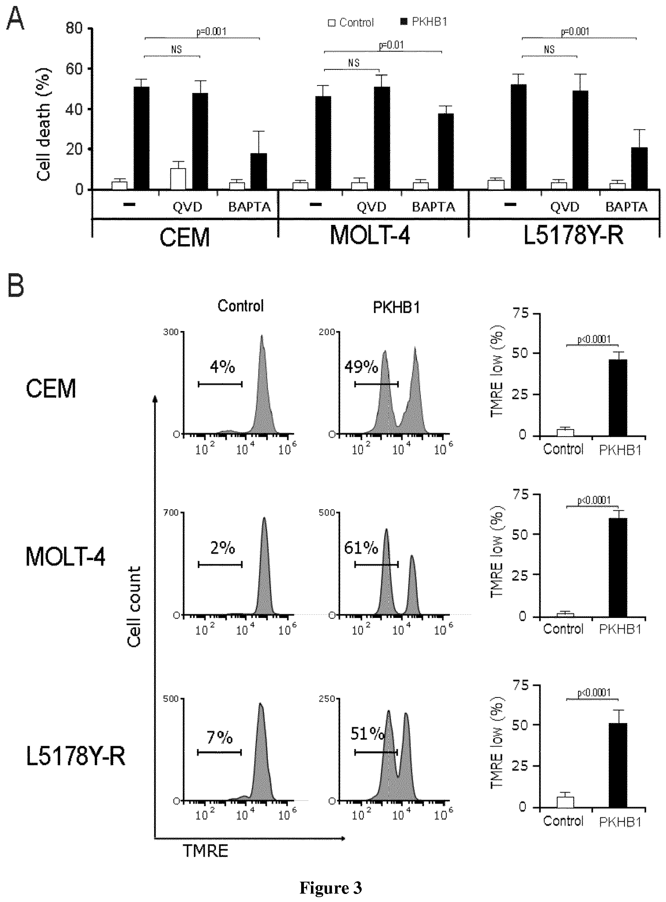

**Figure 3****. PKHB1 induces caspase-independent but calcium-dependent cell death and loss of mitochondrial membrane on leukemia cell lines. A.** Graph represents cell death percentage of T-ALL cells without treatment (Control) or treated with PKHB1 (200µM, 2h) and left alone (-) or pre-incubated for 30min with QVD or Ca2+ chelator BAPTA in the different cell lines tested. **B.** The loss of UΨm induced by PKHB1 (200µM, 2h) was measured in T-ALL cells. Representative cytofluorometric plots are shown.

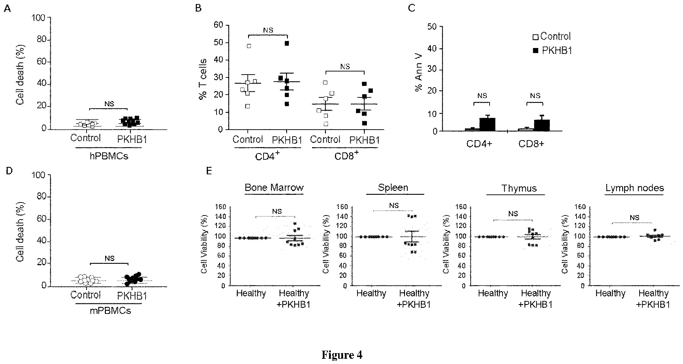

**Figure 4****. PKHB1 spares non-cancerous primary leucocytes from mice and humans in vitro. A.** Cell death of PBMCs treated with PKHB1 was measured by Annexine-V/PI staining (n=10 donors). **B.** Percentage of CD4+, and CD8+ T cells from each donor, left untreated or treated with PKHB1 **C.** Cell death was measured by Annexin-V-APC and graphed. **D.** Cell death of murine PBMCs treated with PKHB1 was measured by Ann/PI staining (n=10). **E.** Cell viability of bone marrow, spleen, thymus and lymph nodes from healthy mice measured by MTT assays n=9 mice.

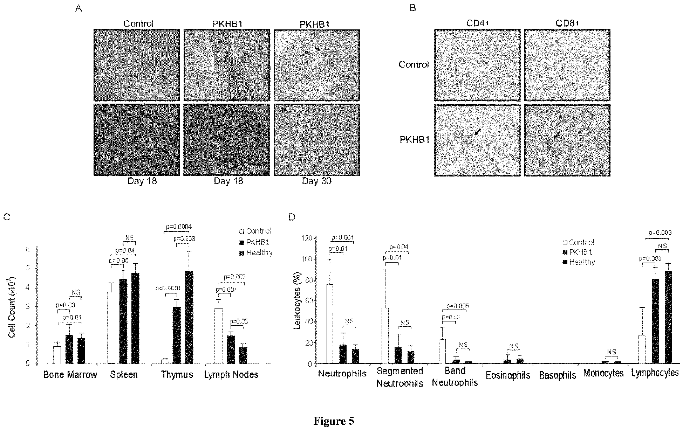

**Figure 5****. PKHB1-treatment of L5178Y-R tumor-bearing mice induces leukocyte infiltration to the tumor site and improves leukocyte-cell number. A.** Histology from tumors from control and PKHB1-treated mice (day 18), stained with H&E. Mitotic cells (red-arrow), lymphocytes (blue-arrow), eosinophils (yellow-arrow), giant cells (blackarrow), necrosis (brown-arrow) and, normal tissue (green-arrow). **B.** For immunohistochemical staining, CD4+ and CD8+ cells were labeled in tumor tissue of control and PKHB1-treated mice. Arrows point cells with positive labeling. **C.** Cell count of lymphoid organs coming from mice with tumor without treatment (Control), mice with tumor treated with PKHB1 or mice without tumor nor treatment (healthy) was performed using trypan blue staining (n=6). **D.** Different types of leukocytes from control, PKHB1-treated and healthy mice are displayed in the graph, obtained using hematic biometry analysis.

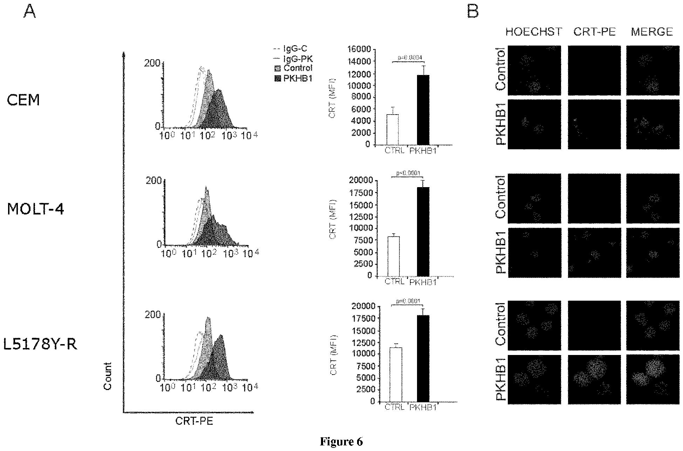

**Figure 6****. PKHB1 induces calreticulin exposure. A.** Left charts are representative of surface CRT detection in CEM (upper), MOLT-4 (middle) and L5178Y-R (bottom) cells using FACS. Negative controls, with IgG isotype antibodies, are shown in dotted (IgG-C) and solid (IgG-PK) lines, while Gray (control) is the basal CRT and black are cells treated (PKHB1). **B.** ECTO-CRT was observed in the cells treated with PKHB1 by CRT-PE staining and nucleus was stained with Hoechst 33342 and visualized by confocal microscopy 40x.

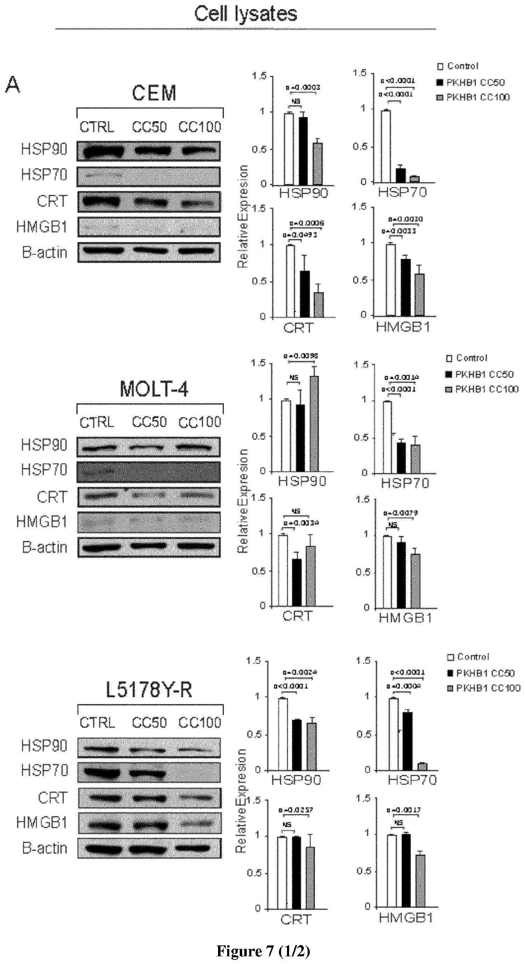

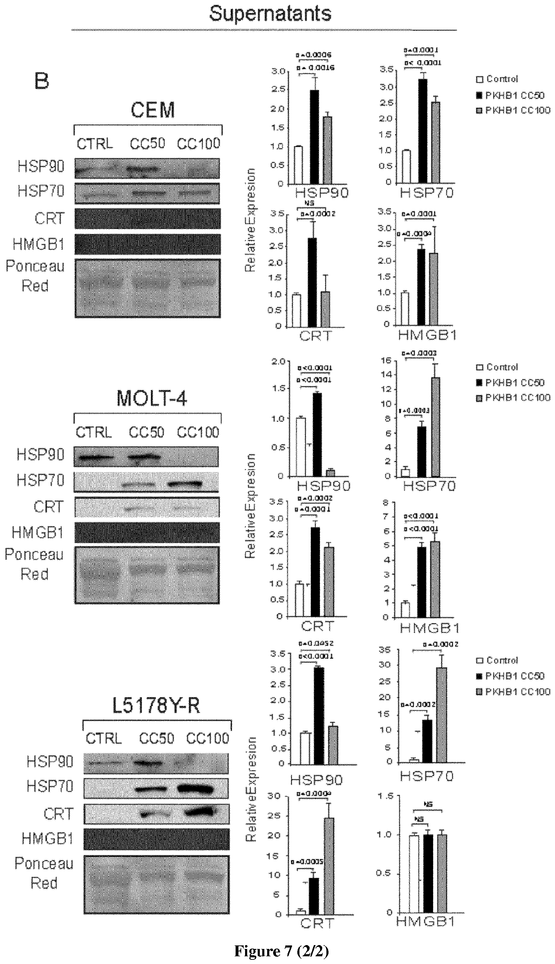

**Figure 7****. HSP90, HSP70, CRT and HMGB1 proteins expression and release in response to treatment with PKHB1.** Western blot and densitometry analyses were performed using cellular lysates **(A)** or supernatants **(B)** of CEM, MOLT-4 and L5178Y-R cells untreated and treated with PKHB1. Loading control was β-actin, and Ponceau red.

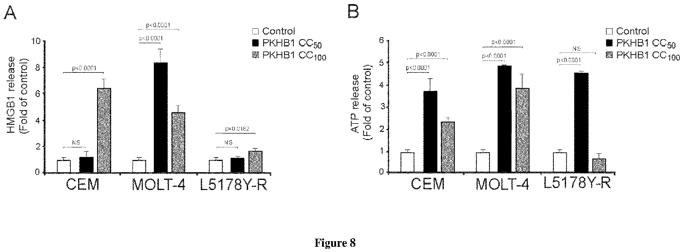

**Figure 8****. PKHB1 induce HMGB1 and ATP release in CEM, MOLT-4 and L5178Y-R cell lines.** Cells were treated with PKHB1 at CC50 and CC100 for 2h, then 100µL of supernatant of each sample was taken to measure the HMGB1 release by ELISA **(A)** or ATP release through bioluminescence detection **(B).** The charts shown are representative of triplicates of three similar experiments.

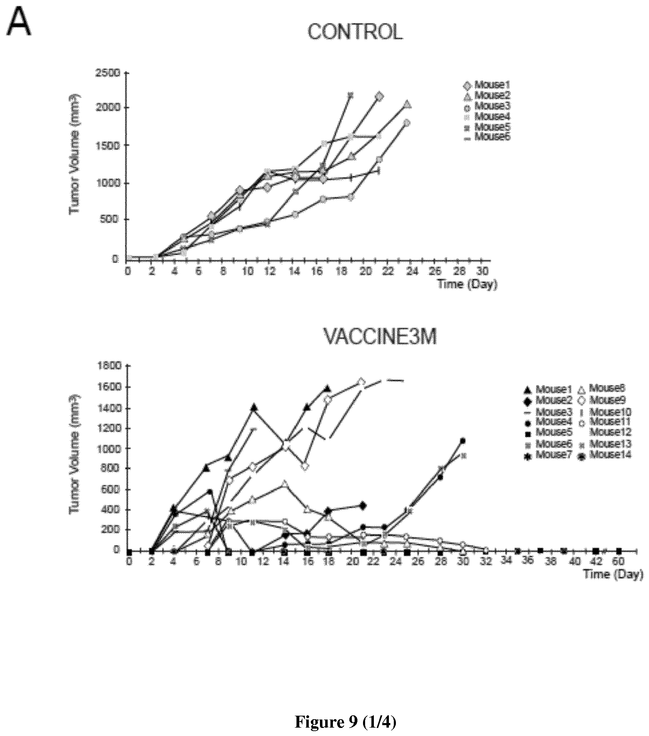

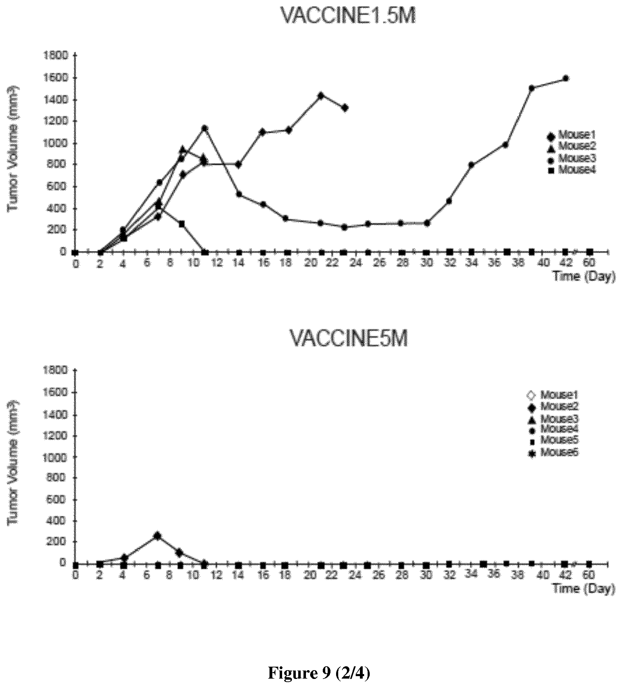

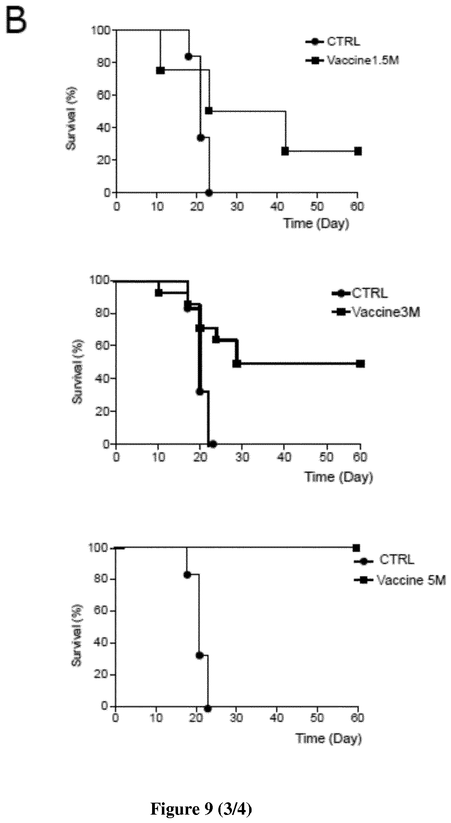

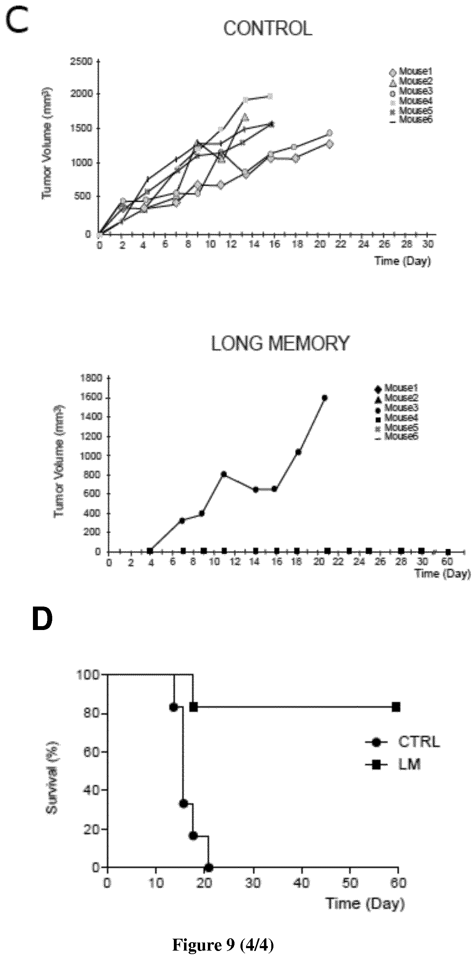

**Figure 9****. PKHB1 induces short- and long-term immunological memory, through prophylactic vaccination or prior exposure to the tumor and treatment. A.** Graphs indicate tumor growth in unvaccinated mice (Control; n=6) or vaccinated with h 1.5x10⁶ (1.5M; n=4), 3x10⁶ (3M; n=8) or 5x106 (5M; n=6) CC100 PKHB1-treated L5178Y-R cells and re-challenged with living L5178Y-R cells. **B.** Survival in vaccinated mice over time. **C.** Long-term anti-tumor memory of mice in remission re-challenged with 2 million L5178YR cells (control n=6, PKHB1-treated n=6). **D.** Survival in re-challenged mice over time. Survival is represented by the Kaplan-Meier graph.

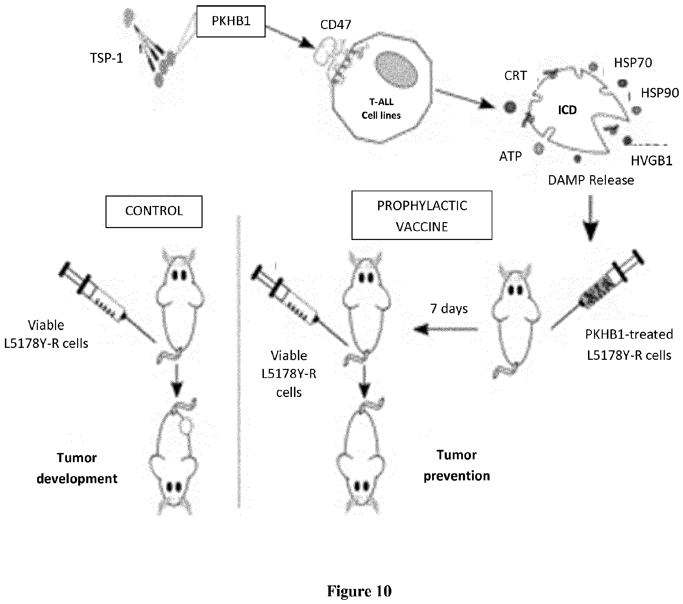

**Figure 10****. Schematic representation of CD47-medited ICD.** PKHB1, induces fast immunogenic cell death in T-ALL cells, leading to DAMP release. The administration of a prophylactic antitumor vaccine, of tumor cells previously treated with PKHB1, prevented tumor establishment *in vivo.*

### EXAMPLES

### I. Material and Methods

### Blood and PBMCs isolation

Peripheral blood was collected from 10 healthy volunteers after obtaining written informed consent. This study was approved by the Institutional Ethics Committee at the Universidad Autonoma de Nuevo Leon, College of Biological Sciences. The animal study was approved by the Animal Ethical Committee (CEIBA), Number: 01/2015. All experiments were conducted according to Mexican regulation NOM-062-ZOO-1999.

The blood from sacrificed mice was obtained by cardiac puncture, while human blood was collected by venipuncture. Peripheral blood mononuclear cells (PBMCs) isolation was performed by density gradient centrifugation using Ficoll-Hypaque-1119 (Sigma-Aldrich, St Louis, MO, USA). 4x10⁵ white blood cells were washed and seeded. CD4+/CD8+ characterization was done using primary antibodies (CD4; MT310 sc-19641 and CD8; 32-M4 sc-1177, Santa Cruz, CA, USA).

### Spleen, Thymus, Lymph Nodes, and Bone Marrow cells extraction

Spleen, thymus, lymphatic node, and bone marrow cells were obtained from female BALB/c mice post-sacrifice. Spleen cells were obtained through perfusion, thymocytes and lymphatic node cells were obtained by maceration, and bone morrow cells (from one femur and tibia per mouse) were flushed with PBS. Cells washed and counted using trypan blue staining.

### Cell culture

CEM, MOLT-4 (T-acute lymphoblastic leukemia, T-ALL), and L5178Y-R (murine lymphoblastic T cell line) were obtained from ATCC. Human and murine PBMCs, human CD4+ and CD8+ T cells, and primary lymphoid organ's cells were obtained from healthy individuals. Cells were maintained in RPMI-1640 medium supplemented with 10% of fetal bovine serum, 2mM L-glutamine, 100 U/mL penicillin-streptomycin (GIBCO by Life Technologies, Grand Island, NY, USA), and incubated at 37oC in a controlled humidified atmosphere with 5% CO₂. Cell count was performed using trypan blue (0.4% Sigma-Aldrich), a Neubauer chamber and an optic microscope (Zeiss Primo Star) as proposed by the ATCC's standard protocols.

### Flow Cytometry. Cell death induction, and inhibition

Annexin-V-allophycocyanin (Ann-V-APC 0.1µg/ml; BD Pharingen, San Jose CA, USA), propidium iodide (PI, 0.5µg/ml Sigma-Aldrich), and tetramethylrhodamine ethyl ester (TMRE, 20nM, Sigma-Aldrich) were used for phosphatidylserine exposure, cell viability, and mitochondrial transmembrane potential (UΨm) quantification, respectively, in a BD AccuryC6 flow cytometer (BD Biosciences) (total population 10,000 cells). Data were analyzed using FlowJo software.

1x10⁶ cells/mL were treated for 2h with PKHB1 (as indicated). For the inhibition assays, calcium chelator, BAPTA (5mM, CalbioChem, Merck, Billerica MA, USA); or the pancaspase inhibitor Q-VD-OPh (QVD, 10µM, BioVision, Milpitas CA, USA); were added 30 min before PKHB1.

### Complete Blood Count (CBC)

The heparinized blood acquired from mice, was assessed using the Automatic Hematology Analyzer KontroLab. Blood smears were performed and fixed with methanol, stained with Wright's, and observed under the microscope to perform differential blood white cells counts.

### Calreticulin exposure

1x10⁶ cells/mL were plated, treated with PKHB1, and incubated for 2h. Cells were harvested, washed, and stained with Calreticulin-PE (FMC-75, Enzo Life Science, Farmingdale, NY, USA) antibody (1:1000) in FACS buffer. After 1h of incubation in darkness at room temperature, cells were washed and resuspended in 100µL of FACS buffer to be assessed by flow cytometry. For confocal microscopy (OlympusX70), poly-Llysine was added 24h to sterile coverslips and 1x10⁶ cells/mL were seeded. PKHB1 was added and incubated for 2h. Then, the cells were stained with Calreticulin-PE antibody (1:500) and Hoechst, incubated 1h, and assessed by confocal microscopy.

### Western Blot

1x10⁶ cells/mL were seeded in a serum-free culture medium and treated with PKHB1 (CC₅₀ and CC₁₀₀ for each cell line) or left alone (Control) for 2h. Supernatants was recovered and lysed with lysis buffer (20mM Tris pH 6.8, 2mM EDTA, 300mM NaCl and SDS 2%).

Protein concentration was measured using the DC Protein Assay kit (Bio-Rad, Hercules, CA, USA) and 50µg of protein were loaded into SDS-PAGE gels. After blotting, nitrocellulose filters were probed with primary antibodies (1:1000) against HMGB1 (HAP46.5: sc-56698), HSP70 (C92F3A-5: sc-66048), HSP90 (F-8: SC-13119) and Calreticulin (F-4: sc373863). Anti-mouse or anti-rabbit -HRP served as secondary antibodies (Santa Cruz Biotechnology, CA, USA). Visualization was performed with ECL substrate system (Thermo Scientific, Waltham, MA, USA).

### ATP release assay

1x10⁶ cells/mL were treated with PKHB1 (CC50 and CC100 for each cell lines) for 2h. Supernatants were used to assess extracellular ATP by a luciferase assay (ENLITEN kit, Promega, Madison WI, USA) following the manufacturer's instructions. Bioluminescence was assessed in a microplate reader (Synergy HT, BioTek, Software Gen5, Winooski, VT, USA) at 560nm.

### HMGB1 release assay

Supernatants of untreated and treated (PKHB1 CC₅₀ and CC₁₀₀ for each cell line) leukemic cells (1x10⁶ cells/mL) were used to measure extracellular HMGB1 using the HMGB1 ELISA kit for CEM, MOLT-4 or L5178Y-R cells (BioAssay ELISA kit Human or mouse respectively, US biological Life Science Salem, MA, USA), following the manufacturer instructions. Absorbance was assessed at 450nm.

### In vivo model

Six-to-eight-week-old BALB/c female mice were maintained in controlled environmental conditions (25°C and 12h light/dark cycle) and were supplied with rodent food (Science diet) and water *ad libitum.*
- Prophylactic vaccinations: L5178Y-R cells (1.5, 3, 5x10⁶) were treated with 300µM of PKHB1 (CC₁₀₀) for 2h. Cell death was confirmed using trypan blue staining and flow cytometry. Treated cells were inoculated subcutaneously in 100µl PBS into the left hind of the leg, whereas 2x10⁶ untreated control cells were inoculated into the right hind 7 days later (42).
- Tumor establishment and measurement: Tumor was established by the subcutaneous injection of 1x106 L5178Y-R cells in 100µL PBS, into the left hind of the leg. Tumor volume and weight were measured three times per week using a caliper (Digimatic Caliper Mitutoyo Corporation, Japan) and a digital scale (American Weigh Scale-600-BLK, USA). When the tumor reached 100mm³ the first PKHB1 injection (200µg) was applied (day 0). Tumor volume was determined with the formula: tumor volume (mm3) = 4π/3*A*B*C. Long-term memory assay: Mice in complete remission after PKHB1 treatment, were rechallenged with 2x10⁶ cells in 100µL of PBS, into the opposite limb, and tumor volume was measured like described above.

### Histology and immunohistochemistry

Tissues and organs were obtained and fixed in 10% neutral formalin, embedded in paraffin, sectioned (5µm thickness) and stained with H&E (Sigma-Aldrich, St Louis, MO, USA).

Histopathological analysis was done by an external veterinarian pathologist (National professional certificate 2593012). Immunohistochemistry was done using the appropriate primary antibody (CD4/CD8) and adding the biotinylated secondary antibody. Finally, hematoxylin-counterstained slides are coverslipped using resin as mounting solution and observed under microscopy.

### Statistical Analysis

Mice were randomly assigned to different groups for all *in vivo* studies. Experiments were repeated three independent times. Mann-Whitney test and two-tailed unpaired Student's *t* test were performed using GraphPad Prism Software (San Diego CA, USA) and presented as mean values}SD. The *p* values were considered significant as follows: *p*<0.05; *p*<0.01 and *p*<0.001.

### II. Results

### CD47 agonist peptide PKHB1, induces cell death in human and murine tumor lymphoblastic T-cell lines.

PKHB1 induces cell death in a concentration-dependent manner, since the cells incubated for 2h with crescent concentrations (100, 200 and 300µM) of PKHB1 show an increase in the number of Ann-V-APC/PI positive CEM (Figure 2A), MOLT-4 (Figure 2B) and L5178Y-R (Figure 2C) cells. The cytotoxic concentration that induces around 50% of cell death (CC50) in CEM is 200µM, in MOLT-4 is 300µM, and in L5178Y-R is 200µM.

### PKHB1 prompts caspase-independent but calcium-dependent cell death with loss of mitochondrial membrane potential in CEM, MOLT-4 and L5178Y-R cells.

It has next been assessed whether PKHB1-induced cell death in T-ALL cells shared the principal biochemical features previously described for CD47-mediated cell death; these include caspase independence (43), a sustained calcium influx and mitochondrial membrane potential (UΨm) loss (33,44). Thus, the cells were pre-incubated with a pan-caspase inhibitor (Q-VD-OPH) or an extracellular Ca2+ chelator (BAPTA) and cell death was tested. Caspase inhibition did not prevent PKHB1-induced killing of CEM (51% ±4 to 48% ±5), MOLT-4 (57% ±4 to 51% ±6), and L5178Y-R (52% ±5 to 49%±3) cells. Nevertheless, extracellular calcium chelation significantly reduced PKHB1-induced cell death in all cases: CEM (51% ±4 to 18% ±11), MOLT-4 (57% ±4 to 38% ±3), and L5178Y-R (52% ±5 to 21% ±8) (Figure 3A). Calcium dependence for death induced by an immobilized anti-CD47 (B6H12) was also corroborated in CEM cells (SF1).

Treatment with the PKHB1 CC50 also showed that it induces loss of UΨm in T-ALL (Figure 3B) being of 49%±5 in CEM, 61%±4 in MOLT-4, and of 51%±8 in L5178Y-R.

### PKHB1-treatment spares non-cancerous primary leucocytes derived from human and mice.

The selectivity of PKHB1 in human PBMCs and CD4+ and CD8+ human T cells from healthy donors has been tested (see Figure 4A,B,C respectively) .

Additionally, PKHB1 selectivity has been tested in murine PBMCs (Figure 4D) and primary cultures of bone marrow (BM), spleen, thymus and lymph nodes of healthy (without tumor nor treatment) BALB/c mice through indirect cell viability analysis (Figure 4E). PKHB1 treatment did not significantly affected cell viability of human- nor murine- non-cancerous cells (Figure 4), even though all organs express CD47 in a similar level to the neoplastic cells (SF2). These results denoted the selectivity of PKHB1 to induce cell death just in malignant cells.

### BALB/c mice L5178Y-R tumor-bearing mice treated with PKHB1 show leukocyte infiltration to the tumor site and improved leukocyte-cell number.

After verifying that PKHB1 treatment did not affect healthy leukocytes *in vitro,* these effects *in vivo* have been assessed. Immunocompetent female BALB/c mice were used to bear L5178Y-R tumor cells, and mice were treated weekly with 200µg of PKHB1 intraperitoneally. After 18 days all controls had to be sacrificed, and some PKHB1-treated mice were randomly selected to be sacrificed for comparison. Tumors were dissected, and their morphological and cellular differences were analyzed (Figure 5A). The control group presented undifferentiated lymphoid cells, presumably L5178Y-R cells, some of them performing mitosis (Figure 5A left). Conversely, tumors in PKHB1-treated mice contained a mixture of lymphocytes and polymorphonuclear cells (PMN) (Figure 5A middle).

Moreover, complete tumor regression in most of the mice was observed at day 30, where histological slides show what seems to be an anti-tumor immune response in the inoculation site (Figure 5A right). Thus, it has been decided to carry out an immunohistochemistry of tumor sections, which indicated the presence of CD4+ and CD8+ cells in PKHB1-treated mice (Figure 5B).

In addition, it has been performed cell counts from lymphoid organs that belonged to control, PKHB1-treated or healthy mice. Noticeably, in PKHB1-treated mice, a significant increase in cell number of BM, spleen and thymus cells, and significant decrease in cell number of lymph nodes were observed (Figure 5C). Moreover, cell number of the same organs in PKHB1-treated mice was similar to that of healthy mice. Additionally, the WBC differential was performed, and showed no significant difference between healthy and PKHB1-treated mice, whereas untreated tumor-bearing mice presented a significant difference from the other two groups in all leukocyte types (Figure 5D). Altogether, the above suggests that PKHB1 improves the anti-tumor immune system of tumor-bearing mice and indicates the possible participation of the immune system in complete tumor regression.

### PKHB1 treatment induces DAMPs' exposure and release in T-ALL cells.

The assessment of the exposure and release of several DAMPs in T-ALL cells has been conducted. In Figure 6 it can be observed that CEM, MOLT-4, and L5178Y-R cells incubated with the CC50 of PKHB1, presented a significant increase in CRT exposure, analyzed by flow cytometry (Figure 6A), and confocal microscopy (Figure 6B).

Then, the expression and release of HSP90, HSP70, CRT and HMGB1 were measured. The presence of these DAMPs was determined by Western blot in cellular lysates and supernatant of untreated cells and PKHB1-treated cells at CC50 and CC100 for each cell line tested. Figure 7A displays the decrease in the expression of HSP90, HSP70, CRT, and HMGB1 in cellular lysates of cells treated with PKHB1. Conversely, the expression of these DAMPs increased in PKHB1-treated supernatants compared with the untreated cells (Figure 7B). These results indicate that PKHB1-treatment prompts the release of heat shock proteins, CRT, and HMGB1 to the extracellular medium.

As HMGB1 release was barely detected by Western Blot, an ELISA assay was performed.

HMGB1 release varied depending on the cell line studied, and the concentration of PKHB1 used. Using PKHB1 CC100, in CEM, MOLT-4 and L5178Y-R cell lines, HMGB1 release was of 6-fold, 4-fold and 2-fold, respectively, compared to the untreated control, while using PKHB1 CC50, MOLT-4 cells HMGB1 release was of 8-fold with respect to the control (Figure 8A).

Another important indicator that immunogenic death is taking place is ATP-release.

Therefore, a bioluminescence assay was performed, finding that in supernatants of PKHB1-treated cells at CC50 and CC100, the presence of ATP significantly augmented (Figure 8B).

### PKHB1-treated cells as prophylactic vaccine prevented the tumor establishment of L5178Y-R cells

Considering the previous data, pointing that PKHB1-treatment induces ICD, the next step was to perform a prophylactic vaccination, which is the gold-standard to confirm whether PKHB1-treatment induced ICD *in vivo.* The vaccine is based in the use of L5178Y-R cells treated *in vitro* with PKHB1 CC₁₀₀. Four groups of mice where used, i. control group without vaccine, ii. 1.5M vaccine group, with 1.5x10⁶ PKHB1-treated cells, iii. 3M vaccine group, with 3x106 PKHB1-treated cells and iv. 5M vaccine group with 5x106 PKHB1-treated cells. The results demonstrated that vaccination containing PKHB1-treated cells prevented the establishment of L5178Y-R tumor, and a greater number of dead cells due to the peptide, depicted better response against tumor cells inoculated 7 days after the vaccine administration (Figure 9). In control group, 6 out of 6 mice (100%) developed the tumor after the inoculation with viable cells (Figure 9A top-left), while 3 out of 4 mice (75%) developed tumor in the 1.5M vaccine group (Figure 7A top-right), 7 out of 14 mice (50%) developed tumor in the 3M vaccine group (Figure 7A down-left), and none of the mice (0%) in the 5M vaccine group developed the tumor (Figure 7A downright). The 60-days survival rates of mice in each group was consistent with tumor growth, being of 100% in the 5M vaccine group (Figure 9B).

### PKHB1-treatment induced long-term prevention of tumor establishment.

Additionally, the long-term tumor prevention in mice that presented complete tumor regression after PKHB1-treatment has been assessed. In these experiments 1 out of 6 mice (≈17%) rechallenged with 2x10⁶ L5178Y-R viable cells developed the tumor, while in the naive control group 6 out of 6 (100%) presented tumor growth (Figure 9C). The survival percentage was graphed using Kaplan-Meier curve, where re-challenged mice presented 90% of survival (Figure 9D).

### III. Discussion

The present assays assessed the ability of PKHB1 peptide, i) to induce selectively cell death in T-ALL cells with the conserved characteristics of CD47-mediated cell death, and ii) to determine if this type of cell death is immunogenic.

It has been observed that PKHB1-induced death in CEM, MOLT-4, and L5178Y-R cells (Figure 2), is a fast caspase-independent process that implicates phosphatidylserine exposure together with plasma membrane permeabilization, and loss of mitochondrial membrane potential (Figure 3) that is selective to malignant cells (Figure 4). In addition, it has been observed that calcium dependence for cell death induced by PKHB1 was conserved in T-ALL cells, as previously observed in CLL cells (33).

These results showed that treatment with PKHB1 into tumor-bearing mice induces leukocyte infiltration to the tumor site and improves leukocyte-cell number in different lymphoid organs (Figure 5). Indeed, PKHB1 was capable to prompt DAMPs exposure and release on T-ALL cells. As CRT is one of the principal molecules shown to be necessary to determine that the cell death is immunogenic (6,18). It is demonstrated its exposure, by flow cytometry and confocal microscopy, on T-ALL after the PKHB1 treatment (Figure 6). Diverse studies in the immunology field highlight the importance of CRT exposure as an "eat me" signal (6,15,46,47) that helps antigen up-take by APCs by binding to low density lipoprotein receptor-related protein 1 (LRP1) (7). There is a tight correlation between CRT and CD47 expression in cancer cells (47). Indeed, recently it was determined that treatment of breast cancer cell lines with thrombospondin (TSP) promoted interaction of TSP with CRT and CD47 and induced cell autophagy and tumor growth inhibition in xenograft mice (48). These results support the idea that TSP or peptides derived from TSP can induce cell death through CD47 activation and its correlation with CRT exposure. Also, HSP70 and HSP90, HMGB1 and ATP were released by PKHB1 treatment on CEM, MOLT-4 and L5178Y-R cell lines (Figure 7 & 8). The release of these molecules is involved in the activation of immune system and induction of potent anticancer immunity (17,49,50). However, DAMPs release is not sufficient to ensure ICD induction, and the *in vivo* vaccination is considered the gold-standard (1,18,21). The *in vivo* assays showed that PKHB1 activates short and long-term immunological memory and induces a protective anti-cancer response in an immunocompetent murine model, since tumor growth was prevented in most cases (Figure 10). Increasing the number of PKHB1-treated cells in the vaccine improves its protective anti-tumor response (Figure 9).

### References

1. Kroemer G, Galluzzi L, Kepp O, Zitvogel L. Immunogenic cell death in cancer therapy. Annu Rev Immunol. 2013; 31:51-72.
2. Galluzzi L, Vitale I, Aaronson SA, Abrams JM, Adam D, Agostinis P, Alnemri ES, Altucci L, Amelio I, Andrews DW, Annicchiarico-Petruzzelli M. Molecular mechanisms of cell death: recommendations of the Nomenclature Committee on Cell Death 2018. Cell Death Differ. 2018; 25(3):486-541.
3. Land WG. The role of damage-associated molecular patterns (DAMPs) in human diseases: part II: DAMPs as diagnostics, prognostics and therapeutics in clinical medicine. Sultan Qaboos University Medical Journal. 2015;15(2): e157.
4. Rubartelli A, Lotze MT. Inside, outside, upside down: damage-associated molecular-pattern molecules (DAMPs) and redox. Trends Immunol. 2007;28(10):429-36.
5. Garg AD, Dudek AM, Agostinis P. Cancer immunogenicity, danger signals, and DAMPs: what, when, and how?. Biofactors. 2013;39(4):355-67.
6. Obeid M, Tesniere A, Ghiringhelli F, Fimia GM, Apetoh L, Perfettini JL, Castedo M, Mignot G, Panaretakis T, Casares N, Metivier D. Calreticulin exposure dictates the immunogenicity of cancer cell death. Nat Med. 2007;13(1):54-61.
7. Garg AD, Krysko DV, Verfaillie T, Kaczmarek A, Ferreira GB, Marysael T, et al. A novel pathway combining calreticulin exposure and ATP secretion in immunogenic cancer cell death. EMBO J. 2012;31(5):1062-79.
8. Fucikova J, Kasikova L, Truxova I, Laco J, Skapa P, Ryska A, et al. Relevance of the chaperone-like protein calreticulin for the biological behavior and clinical outcome of cancer. Immunol Lett. 2018; 193:25-34.
9. Spisek R, Charalambous A, Mazumder A, Vesole DH, Jagannath S, Dhodapkar MV. Bortezomib enhances dendritic cell (DC)-mediated induction of immunity to human myeloma via exposure of cell surface heat shock protein 90 on dying tumor cells: therapeutic implications. Blood. 2007;109(11):4839-45.
10. Garg AD, Galluzzi L, Apetoh L, Baert T, Birge RB, Pedro BS, et al. Molecular and translational classifications of DAMPs in immunogenic cell death. Front Immunol. 2015;6:588.
11. Elliott MR, Chekeni FB, Trampont PC, Lazarowski ER, Kadl A, Walk SF, et al. Nucleotides released by apoptotic cells act as a find-me signal to promote phagocytic clearance. Nature. 2009;461(7261):282.
12. Aymeric L, Apetoh L, Ghiringhelli F, Tesniere A, Martins I, Kroemer G, et al. Tumor cell death and ATP release prime dendritic cells and efficient anticancer immunity. Cancer Res. 2010;70(3):855-8.
13. Martins I, Wang Y, Michaud M, Ma Y, Sukkurwala AQ, Shen S, et al. Molecular mechanisms of ATP secretion during immunogenic cell death. Cell Death Differ 2014;21(1):79-9175.
14. Scaffidi P, Misteli T, Bianchi ME. Release of chromatin protein HMGB1 by necrotic cells triggers inflammation. Nature. 2002;418(6894):191-5.
15. Inoue H, Tani K. Multimodal immunogenic cancer cell death as a consequence of anticancer cytotoxic treatments. Cell death and differentiation. 2014;21(1):39-49.
16. Tesniere A, Panaretakis T, Kepp O, Apetoh L, Ghiringhelli F, Zitvogel L, et al. Molecular characteristics of immunogenic cancer cell death. Cell death and Differ. 2008;15(1):3-12.
17. Krysko DV, Garg AD, Kaczmarek A, Krysko O, Agostinis P, Vandenabeele P. Immunogenic cell death and DAMPs in cancer therapy. Nature Reviews Cancer. 2012;12(12):860.
18. Bezu L, Gomes-da-Silva LC, Dewitte H, Breckpot K, Fucikova J, Spisek R, et al. Combinatorial strategies for the induction of immunogenic cell death. Front Immunol. 2015;6:187.
19. Kepp O, Senovilla L, Vitale I, Vacchelli E, Adjemian S, Agostinis P, et al. Consensus guidelines for the detection of immunogenic cell death. Oncoimmunology. 2014;3(9):e955691.
20. Garg AD, More S, Rufo N, Mece O, Sassano ML, Agostinis P, et al. Trial Watch: Immunogenic cell death induction by anticancer chemotherapeutics. OncoImmunology. 2017;6(12):e1386829.
21. Galluzzi L, Buque A, Kepp O, Zitvogel L, Kroemer G. Immunogenic cell death in cancer and infectious disease. Nat Rev Immunol. 2017;17(2):97-111
22. Jabbour E, O'brien S, Konopleva M, Kantarjian H. New insights into the pathophysiology and therapy of adult acute lymphoblastic leukemia. Cancer. 2015;121(15):2517-28.
23. Terwilliger T, Abdul-Hay M. Acute lymphoblastic leukemia: a comprehensive review and 2017 update. Blood Cancer J. 2017;7(6):e577.
24. National Cancer Institute. SEER cancer statistics review, 1975-2015. Leukemia, annual incidence rates (acute lymphocytic leukemia). https://seer.cancer.gov/statfacts/html/alyl.html
25. McNeer J.L., Bleyer A., Conter V., Stock W. Acute Lymphoblastic Leukemia. In: Bleyer A., Barr R., Ries L., Whelan J., Ferrari A. (eds) Cancer in Adolescents and Young Adults. Pediatric Oncology. Springer, Cham. 2017; 151-175
26. McNeer JL, Bleyer A. Acute lymphoblastic leukemia and lymphoblastic lymphoma in adolescents and young adults. Pediatric Blood & Cancer. 2018;65(6):e26989.
27. Jaime-Perez JC, Fernandez LT, Jimenez-Castillo RA, Gomez-De Leon A, Cantu-Rodriguez OG, Gutierrez-Aguirre CH, et al. Age Acts as an Adverse Independent Variable for Survival in Acute Lymphoblastic Leukemia: Data From a Cohort in Northeast Mexico. Clin Lymphoma, Myeloma Leuk. 2017;17(9):590-4.
28. Li Y, Buijs-Gladdines JG, Cante-Barrett K, Stubbs AP, Vroegindeweij EM, Smits WK, et al. IL-7 receptor mutations and steroid resistance in pediatric t cell acute lymphoblastic leukemia: A Genome Sequencing Study. PLoS medicine. 2016;13(12):e1002200.
29. Locatelli F, Schrappe M, Bernardo ME, Rutella S. How I treat relapsed childhood acute lymphoblastic leukemia. Blood. 2012;120(14):2807-16.
30. Pogorzala M, Kubicka M, Rafinska B, Wysocki M, Styczynski J. Drug-resistance profile in multiple-relapsed childhood acute lymphoblastic leukemia. Anticancer Res. 2015;35(10):5667-70.
31. Thompson PA, Tam CS, O'Brien SM, Wierda WG, Stingo F, Plunkett W, et al. Fludarabine, cyclophosphamide, and rituximab treatment achieves long-term disease-free survival in IGHV-mutated chronic lymphocytic leukemia. Blood. 2016;127(3):303-9.
32. Majeti R, Chao MP, Alizadeh AA, Pang WW, Jaiswal S, Gibbs Jr KD, et al. CD47 is an adverse prognostic factor and therapeutic antibody target on human acute myeloid leukemia stem cells. Cell. 2009;138(2):286-99.
33. Martinez-Torres AC, Quiney C, Attout T, Boullet H, Herbi L, Vela L, et al. CD47 agonist peptides induce programmed cell death in refractory chronic lymphocytic leukemia B cells via PLCγ1 activation: evidence from mice and humans. PLoS medicine. 2015;12(3):e1001796.
34. Denefle T, Boullet H, Herbi L, Newton C, Martinez-Torres AC, Guez A, et al. Thrombospondin-1 Mimetic Agonist Peptides Induce Selective Death in Tumor Cells: Design, Synthesis, and Structure-Activity Relationship Studies. J. Med Chem. 2016;59(18):8412-21.
35. Chao MP, Alizadeh AA, Tang C, Jan M, Weissman-Tsukamoto R, Zhao F, et al. Therapeutic antibody targeting of CD47 eliminates human acute lymphoblastic leukemia. Cancer Res. 2011 Feb 15;71(4):1374-84.
36. Barclay AN, van den Berg TK. The interaction between signal regulatory protein alpha (SIRPα) and CD47: structure, function, and therapeutic target. Annu Rev Immunol. 2014;32:25-50.
37. Soto-Pantoja DR, Kaur S, Roberts DD. CD47 signaling pathways controlling cellular differentiation and responses to stress. Critical Rev Biochem Mol Biol. 2015;50(3):212-30.
38. Liu X, Pu Y, Cron K, Deng L, Kline J, Frazier WA, et al. CD47 blockade triggers T cell-mediated destruction of immunogenic tumors. Nat Med. 2015;21(10):1209-15.
39. Leclair P, Liu CC, Monajemi M, Reid GS, Sly LM, Lim CJ. CD47-ligation induced cell death in T-acute lymphoblastic leukemia. Cell death Dis. 2018;9(5).
40. Mateo V, Lagneaux L, Bron D, Biron G, Armant M, Delespesse G, et al. CD47 ligation induces caspase-independent cell death in chronic lymphocytic leukemia. Nat Med. 1999;5(11):1277-84.
41. Sick E, Jeanne A, Schneider C, Dedieu S, Takeda K, Martiny L. CD47 update: a multifaceted actor in the tumour microenvironment of potential therapeutic interest. Br J Pharmacol. 2012;167(7):1415-30.
42. Casares N, Pequignot MO, Tesniere A, Ghiringhelli F, Roux S, Chaput N, et al. Caspase-dependent immunogenicity of doxorubicin-induced tumor cell death. J Exp Med. 2005;202(12):1691-701.
43. Oldenborg PA. CD47: a cell surface glycoprotein which regulates multiple functions of hematopoietic cells in health and disease. ISRN Hematol. 2013;2013:1-19.
44. Manna PP, Frazier WA. The mechanism of CD47-dependent killing of T cells: heterotrimeric Gi-dependent inhibition of protein kinase A. J Immunol. 2003;170(7):3544-53.
45. Zhou H, Forveille S, Sauvat A, Yamazaki T, Senovilla L, Ma Y, et al. The oncolytic peptide LTX-315 triggers immunogenic cell death. Cell Death Dis. 2016;7(3):e2134.
46. Gardai SJ, McPhillips KA, Frasch SC, Janssen WJ, Starefeldt A, Murphy-Ullrich JE, et al. Cell-surface calreticulin initiates clearance of viable or apoptotic cells through transactivation of LRP on the phagocyte. Cell. 2005;123(2):321-34.
47. Chao MP, Jaiswal S, Weissman-Tsukamoto R, Alizadeh AA, Gentles AJ, Volkmer J. et al. Calreticulin is the dominant pro-phagocytic signal on multiple human cancers and is counterbalanced by CD47. Sci Transl Med. 2010;2(63):63-94
48. Chen Q, Fang X, Jiang C, Yao N, Fang X. Thrombospondin promoted anti-tumor of adenovirus-mediated calreticulin in breast cancer: Relationship with anti-CD47. Biomed Pharmacother. 2015;73:109-15.
49. Fucikova J, Kralikova P, Fialova A, Brtnicky T, Rob L, Bartunkova J, et al. Human tumor cells killed by anthracyclines induce a tumor-specific immune response. Cancer Res. 2011;71(14):4821-33.
50. Rodriguez-Salazar MD, Franco-Molina MA, Mendoza-Gamboa E, Martinez-Torres AC, Zapata-Benavides P, Lopez-Gonzalez JS, et al. The novel immunomodulatory IMMUNEPOTENT CRP combined with chemotherapy agent increased the rate of immunogenic cell death and prevented melanoma growth. Oncol Lett. 2017;14(1):844-52.
51. Dudek-Perić AM, Ferreira GB, Muchowicz A, Wouters J, Prada N, Martin S, et al. Antitumor immunity triggered by melphalan is potentiated by melanoma cell surface-associated calreticulin. Cancer Res. 2015;75(8):1603-14.
52. Guo C, Manjili MH, Subjeck JR, Sarkar D, Fisher PB, Wang XY. Therapeutic cancer vaccines: past, present, and future. Adv Cancer Res 2013;119:421-75).
53. Showalter A, Limaye A, Oyer JL, Igarashi R, Kittipatarin C, Copik AJ, et al. Cytokines in immunogenic cell death: Applications for cancer immunotherapy. Cytokine. 2017;97:123-32.
54. Papaioannou NE, Beniata OV, Vitsos P, Tsitsilonis O, Samara P. Harnessing the immune system to improve cancer therapy. Ann Transl Med. 2016;4(14) :261-261.
55. Li X. The inducers of immunogenic cell death for tumor immunotherapy. Tumori J 2018;104(1): 1-8.
56. Pol J, Vacchelli E, Aranda F, Castoldi F, Eggermont A, Cremer I, et al. Trial Watch: Immunogenic cell death inducers for anticancer chemotherapy. Oncoimmunology. 2015;4(4):e1008866.
57. Vacchelli E, Aranda F, Eggermont A, Galon J, Sautes-Fridman C, Zitvogel L, et al. Trial Watch: Tumor-targeting monoclonal antibodies in cancer therapy. Oncoimmunology. 2014;3(1):e27048.
58. Tanaka M, Kataoka H, Yano S, Sawada T, Akashi H, Inoue M, et al. Immunogenic cell death due to a new photodynamic therapy (PDT) with glycoconjugated chlorin (G-chlorin). Oncotarget. 2016;7(30):47242-51.
59. Golden EB, Apetoh L. Radiotherapy and immunogenic cell death. Semin Radiat Oncol. 2015;25(1):11-7
60. Diaconu I, Cerullo V, Hirvinen ML, Escutenaire S, Ugolini M, Pesonen SK, et al. Immune response is an important aspect of the antitumor effect produced by a CD40L-encoding oncolytic adenovirus. Cancer Res. 2012;72(9):2327-38.
61. Yamano T, Kubo S, Fukumoto M, Yano A, Mawatari-Furukawa Y, Okamura H, et al. Whole cell vaccination using immunogenic cell death by an oncolytic adenovirus is effective against a colorectal cancer model. Mol Ther-Oncolytics. 2016;3:16031.
62. Fucikova J, Moserova I, Truxova I, Hermanova I, Vancurova I, Partlova S, et al. High hydrostatic pressure induces immunogenic cell death in human tumor cells. Int J Cancer. 2014;135(5):1165-77.
63. Lin TJ, Lin HT, Chang WT, Hsiao PW, Yin SY, et al. Shikonin-enhanced cell immunogenicity of tumor vaccine is mediated by the differential effects of DAMP components. Mol Cancer. 2015;14(1):174.
64. Yin S, Yang NS, Lin TJ. Molecular basis of shikonin-induced immunogenic cell death: insights for developing cancer therapeutics. Recept Clin Investig. 2016;3:1-5.
65. D'Eliseo D, Manzi L, Velotti F. Capsaicin as an inducer of damage-associated molecular patterns (DAMPs) of immunogenic cell death (ICD) in human bladder cancer cells. Cell Stress and Chaperones. 2013;18(6):801-8.
66. Jin T, Wu H, Wang Y, Peng H. Capsaicin induces immunogenic cell death in human osteosarcoma cells. Exp Ther Med. 2016;12(2):765-70.

### Sequence listing

KRFYVVMWKK (SEQ ID NO: 1).
PKHB1: (D)K-R-F-Y-V-V-M-K-(D)K; (SEQ. ID. N°2)
Ac-RFYVVMWK-NH₂ (SEQ. ID. N°3)
Ac-KRFYVVMWKK-NH₂ (SEQ. ID. N°4)
H-(D)KAFYVVMWK(D)K-OH (SEQ. ID. N°5)
H-(D)KRFYVV(Nle)WK(D)K-OH (SEQ. ID. N°6)
H-FYVVXW-OH (SEQ. ID. N°7)
H-FYVVXW-NH₂ (SEQ. ID. N°8)
Ac-FYVVXW-OH (SEQ. ID. N°9)
Ac-FYVVXW-NH₂ (SEQ. ID. N°10)
H-(D)KFYVVXW(D)K-OH (SEQ. ID. N°11)
H-FYVVKW-OH (SEQ. ID. N°12)
H-FYVVKW-NH₂ (SEQ. ID. N°13)
H-(D)K ψ(CONMe)R F Y V V M W K (D)K-OH (SEQ. ID. N°14)
H-(D)K R F Y V V M W ψ(CONMe)K (D)K-OH (SEQ. ID. N°15)
H-(D)K ψ(CONMe)R F Y V V M W ψ(CONMe)K (D)K-OH (SEQ. ID. N°16)
H-(D)K ψ(CONMe)R F Y V V X W K (D)K-OH (SEQ. ID. N°17, PKT16)
H-(D)K ψ(CONMe)R F Y V V L W K (D)K-OH (SEQ. ID. N°18)
H-(D)K ψ(CONMe)R F Y VV I W K (D)K-OH (SEQ. ID. N°19)
H-(D)K ψ(CONMe)R F F V V X W K (D)K-OH (SEQ. ID. N°20)

| **PEPTIDES** | **AMINO ACID SEQUENCE (linear representation)** |
|---|---|
| PKD10 (SEQ. ID. N°21) | -F-Y-V-V-K-W-p-P-L-S-V-K-V-V- |
| PKD10FF (SEQ. ID. N°22) | -F-F-V-V-K-W-p-P-L-S-V-K-V-V- |
| PKTDi2 (SEQ. ID. N°23) | -p-P-R-F-Y-V-V-M-W-K-G-L-S-V-K-V-V-N- |
| PKTD1 (SEQ. ID. N°24) | -S-R-F-Y-V-V-M-W-K-p-P-G-I-S-V-K-V-V-K-S- |
| PKTD10 (SEQ. ID. N°25) | -S-R-F-Y-V-V-M-W-K-p-P-G-L-S-V-K-V-V-N-S- |
| PKTD10-RNMe (SEQ. ID. N°26) | -S-R*-F-Y-V-V-M-W-K-p-P-G-L-S-V-K-V-V-N-S- R* = RNMe |
| PKTD10-X-RNMe (SEQ. ID. N°27) | -S-R*-F-Y-V-V-X-W-K-p-P-G-L-S-V-K-V-V-N-S- R* = RNMe and X = NLe |
| PKTD10-3-X-RNMe (SEQ. ID. N°28) | -S-R*-F-Y-V-V-X-W-K-p-P-G-L-A-V-K-V-V-N-S- R* = RNMe and X =N Le |
| PKTD16 (SEQ. ID. N°29) | -S-R-F-Y-V-V-M-W-K-p-P-S-R-F-Y-V-V-M-W-K- |
| PKTD18 (SEQ. ID. N°30) | -S-R-F-Y-V-V-M-W-K-p-P-G-L-S-V-K-V-V-N-G- |
| PKTD11 (SEQ. ID. N°31) | -S-R-F-Y-V-V-M-W-K-Q-p-P-S-G-L-S-V-K-V-V-N-S- |
| PKTD11-RNMe (SEQ. ID. N°32) | -S-R*-F-Y-V-V-M-W-K-Q-p-P-S-G-L-S-V-K-V-V-N-S- R* = RNMe |
| PKTD11-X-RNMe (SEQ. ID. N°33) | -S-R*-F-Y-V-V-X-W-K-Q-p-P-S-G-L-S-V-K-V-V-N-S- R* = RNMe and X = NLe |

## Claims

1. Synthetic TSP-1-derived peptide for its use to induce immunogenic cell death in the treatment of cancer.

2. Synthetic TSP-1-derived peptide for its use according to claim 1, wherein said synthetic TSP-1-derived peptide is a compound or a pharmaceutical acceptable salt thereof comprising a hexapeptide sequence of formula (I):
-X₁-X₂-X₃-X₄-X₅-X₆- (I)
wherein:
- X₁, X₂, X₃, X₄, X₅, X₆ are independently linked to each other according to formula (I) via peptide bonds or at least one pseudopeptide bond;
- X₁ is a residue chosen in the list consisting of substituted or unsubstituted phenylalanine, substituted or unsubstituted para-tyrosine, substituted or unsubstituted ortho-tyrosine, substituted or unsubstituted meta-tyrosine, or substituted or unsubstituted homo-phenylalanine;
- X₂ is a residue chosen in the list consisting of substituted or unsubstituted para- tyrosine, substituted or unsubstituted ortho-tyrosine, substituted or unsubstituted meta- tyrosine, substituted or unsubstituted phenylalanine, homo-phenylalanine, homo-meta- tyrosine, homo-para-tyrosine or homo-ortho-tyrosine;
- X₃ is a residue chosen in the list consisting of substituted or unsubstituted valine, substituted or unsubstituted alanine, substituted or unsubstituted leucine, substituted or unsubstituted isoleucine, preferably valine;
- X₄ is a residue chosen in the list consisting of substituted or unsubstituted valine, substituted or unsubstituted alanine, substituted or unsubstituted leucine, substituted or unsubstituted isoleucine, preferably valine;
- X₅ is a residue chosen in the list consisting of substituted or unsubstituted methionine or any amino acid with similar properties such as a methylated homo-cysteine, lysine, norleucine, leucine or isoleucine;
- X₆ is a residue chosen in the list consisting of substituted or unsubstituted tryptophan, substituted or unsubstituted hetero-tryptophan, substituted or unsubstituted para-tyrosine, substituted or unsubstituted ortho-tyrosine, substituted or unsubstituted meta-tyrosine, substituted or unsubstituted phenylalanine, or substituted or unsubstituted naphthyl-alanine;
- X₁ is the N-terminal side of the molecule of formula (I), X₆ is the C-terminal side of the molecule of formula (I).

3. Synthetic TSP-1-derived peptide for its use according to claim 1 or claim 2, wherein said peptide is selected amongst PKHB1 (SEQ. ID. N°2), PKT16 (SEQ. ID. N°17) and PKTD10 (SEQ. ID. N°43).

4. Synthetic TSP-1-derived peptide for its use according to anyone of claim 1 to 3, wherein cancer is selected form the group consisting of adrenal cortical cancer, anal cancer, bile duct cancer, multiple myeloma, bladder cancer, bone cancer, brain and central nervous system cancer, breast cancer, Castleman disease, cervical cancer, colorectal cancer, endometrial cancer, esophagus cancer, gallbladder cancer, gastrointestinal carcinoid tumors, Hodgkin's disease, non-Hodgkin's lymphoma, Kaposi's sarcoma, kidney cancer, laryngeal and hypopharyngeal cancer, leukemia, liver cancer, lung cancer, mesothelioma, plasmacytoma, nasal cavity and paranasal sinus cancer, nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma, salivary gland cancer, skin cancer, melanoma, stomach cancer, testicular cancer, thymus cancer, thyroid cancer, vaginal cancer, vulvar cancer, and uterine cancer.

5. *In vitro* use of synthetic TSP-1-derived peptide for inducing immunogenic cell death in tumour cell.

6. Process for preparation of such tumour cell comprising a step of treating said cells with a synthetic TSP-1-derived peptide.

7. Tumour cell treated with a synthetic TSP-1-derived peptide for its use to induce immunogenic cell death for the treatment of cancer.

8. Injectable pharmaceutical composition comprising a tumour cell treated with a synthetic TSP-1-derived peptide and a pharmaceutically acceptable carrier.
